# EUROPEAN PATENT SPECIFICATION

(11) **EP 3 849 539 B1**
(45) Date of publication and mention of the grant of the patent: **01.07.2026**
(21) Application number: 19773718.2
(22) Date of filing: 12.09.2019
(51) Int. Cl.: A61K 31/341, A61K 31/343, A61K 31/401, A61K 31/4166, A61K 45/00, A61P 17/00, A61P 17/02

(54) **PLASMINOGEN ACTIVATOR INHIBITOR 1 (PAI-1) INHIBITORS AND USES THEREFOR**
PLASMINOGEN-AKTIVATOR-INHIBITOR 1 (PAI-1)-INHIBITOREN UND IHRE VERWENDUNGEN
INHIBITEURS DE L'INHIBITEUR DE L'ACTIVATEUR DU PLASMINOGÈNE 1 (PAI-1) ET UTILISATIONS ASSOCIÉES

(30) Priority: 13.09.2018 US 201862731074 P
(43) Date of publication of application: 21.07.2021
(60) Divisional application: 26181362.0
(73) Proprietor: Eirion Therapeutics, Inc., Woburn, Massachusetts 01801 (US)
(72) Inventor: EDELSON, Jonathan, Woburn, Massachusetts 01801 (US)
(74) Representative: J A Kemp LLP
(86) International application number: PCT/US2019/050849
(87) International publication number: WO 2020/056160

(56) References cited:
- WO-A1-03/071267
- US-A1- 2004 043 026
- YAMAOKA NAGAHISA ET AL: "Identification of novel plasminogen activator inhibitor-1 inhibitors with improved oral bioavailability: Structure optimization of N-acylanthranilic acid derivatives", BIOORGANIC & MEDICINAL CHEMISTRY LETTERS, vol. 28, no. 4, 1 February 2018 (2018-02-01), Amsterdam NL, pages 809 - 813, XP055875056, ISSN: 0960-894X, DOI: 10.1016/j.bmcl.2017.11.016
- VERONICA R. PLACENCIO ET AL: "Small Molecule Inhibitors of Plasminogen Activator Inhibitor-1 Elicit Anti-Tumorigenic and Anti-Angiogenic Activity", PLOS ONE, vol. 10, no. 7, 24 July 2015 (2015-07-24), pages e0133786, XP055321128, DOI: 10.1371/journal.pone.0133786
- G. RANIERI ET AL: "Defibrotide in the treatment of Raynaud's phenomenon in patients with progressive systemic sclerosis or essential mixed cryoglobulinemia", CURRENT THERAPEUTIC RESEARCH, vol. 53, no. 1, 1 January 1993 (1993-01-01), pages 48 - 58, XP055044382, ISSN: 0011-393X, DOI: 10.1016/S0011-393X(05)80155-9
- FARHATULLAH SYED ET AL: "Ex vivo evaluation of antifibrotic compounds in skin scarring: EGCG and silencing of PAI-1 independently inhibit growth and induce keloid shrinkage", LABORATORY INVESTIGATION, vol. 93, no. 8, 8 July 2013 (2013-07-08), The United States and Canadian Academy of Pathology, Inc., pages 946 - 960, XP055537724, ISSN: 0023-6837, DOI: 10.1038/labinvest.2013.82

## Description

### Cross-Reference To Related Applications

This application claims priority to United States Provisional Application No. 62/731,074, filed September 13, 2018.

### Background

Graying hair and other dermatological conditions often promote significant anxiety; many people go to great lengths to hide their gray, particularly if it arrives prematurely. Previously available therapies have largely proven unsatisfactory, hence new treatment options are needed.

US 2004/043026 describes the treatment and prevention of abnormal scar formation in keloids and other cutaneous or internal wounds or lesions by reducing the activity of plasminogen activator inhibitor-1 (PAI-1).

WO 03/071267 describes therapeutic methods employing PAI-1 inhibitors and transgenic non-human animal for screening candidate PAI-1 inhibitors.

G. Ranieri et al., Current Therapeutic Research, vol. 53, no. 1 (1993), pages 48-58, describes the use of defibrotide in the treatment of Raynaud's phenomenon in patients with progressive systemic sclerosis or essential mixed cryoglobulinemia.

Farhatullah Syed at al., Laboratory Investigation, vol. 93, no. 8 (2013), pages 946-960, describes a study in which (-)-epigallocatechin-3-gallate and silencing of PAI-1 are found to inhibit growth and induce shrinkage of human keloid tissue.

Nagahisa Yamaoka et al., Bioorganic & Medicinal Chemistry Letters, vol. 28, no. 4 (2018), pages 809-813, describes the identification of PAI-1 inhibitors with improved oral bioavailability by structure-activity relationship studies on N-acyl-5-chloroanthranilic acid derivatives.

Veronica R. Placencio et al., PLOS ONE, vol. 10, no. 7 (2015), e0133786, describes a study of the anti-tumorigenic and anti-angiogenic activity of small molecule PAI-1 inhibitors.

### Summary

The present disclosure provides, *inter alia,* new technologies (e.g., methods, kits, compositions, etc) for treatment and/or prevention of certain dermatological conditions, specifically including graying hair. Among other things, the present disclosure provides an insight that plasminogen activator inhibitor -1 (PAI-1) inhibitors may be useful in the treatment and/or prevention of certain dermatological conditions, for example, in treatment and/or prevention of graying hair. The aspects of the disclosure that constitute the present invention are set out in the appended set of claims.

The present invention accordingly provides a non-therapeutic cosmetic process for treating or preventing hair graying, the process comprising providing a composition that comprises a plasminogen activator inhibitor-1 (PAI-1) inhibitor and: (i) administering the composition to a site of a subject, wherein the site contains or did contain a plurality of hair follicles, each with a hair disposed therein, so that the PAI-1 inhibitor is delivered to the subject; or (ii) administering the composition to a subject, wherein a site of the subject contains or did contain a plurality of hair follicles, each with a hair disposed therein, so that the PAI-1 inhibitor is delivered to the subject; wherein the PAI-1 inhibitor is selected from 5-Chloro-2-{[(2-{[3-(furan-3-yl)phenyl]amino}-2-oxoethoxy)acetyl]amino benzoic acid, and 5-Chloro-2-{[{[3-(furan-3-yl)phenyl]amino}(oxo)acetyl]amino} benzoic acid.

Those skilled in the art are aware that over-expression of PAI-1 is associated with the prevention of the conversion of plasminogen to plasmin which is essential to fibrinolysis, which is the physiological breakdown of blood clots. The present disclosure provides an insight PAI-1 may also be associated with certain dermatological conditions. The present disclosure provides new technologies (e.g., methods, kits, compositions, etc) for treatment and/or prevention of certain dermatological conditions, including graying hair. Alternatively or additionally, in some embodiments, provided technologies may be utilized for treatment and/or prevention of dermatological conditions such as keloids, scleroderma, and Raynaud's phenomenon.

In some embodiments, the present disclosure provides methods of treating a dermatological condition comprising providing a composition that comprises or delivers a plasminogen activator inhibitor-1 (PAI-1) inhibitor; administering the composition to a site of a subject, wherein the site contains or did contain a plurality of hair follicles, each with a hair disposed therein, so that the PAI-1 inhibitor is delivered to the subject. Additionally or alternatively, in some embodiments, the present disclosure provides methods of treating a dermatological condition comprising administering a provided composition to a subject, at a site of the subject contains or did contain a plurality of hair follicles, each with a hair disposed therein, so that the PAI-1 inhibitor is delivered to the subject.

In some embodiments, the present disclosure provides methods of preventing the occurrence or progression of a dermatological condition, comprising providing a composition that comprises or delivers a plasminogen activator inhibitor-1 (PAI-1) inhibitor; administering the composition topically to a site of the subject, wherein the site is a skin surface that contains or contained a plurality of hair follicles, each with a hair disposed therein; and leaving the composition on the site for a period of time, so that the PAI-1 inhibitor is delivered to the subject.

In some embodiments, the present disclosure provides new technologies for treating and/or preventing the occurrence or progression of a dermatological condition. In some embodiments, a dermatological condition is or comprises hair graying. In some embodiments, a dermatological condition is or comprises keloids. In some embodiments, a dermatological condition is or comprises scleroderma. In some embodiments, a dermatological condition is or comprises Raynaud's disease.

In some embodiments, the present disclosure provides new compositions that comprise and/or deliver a therapeutically effective amount of a PAI-1 inhibitor. In certain embodiments, the present disclosure provides new compositions that comprise and/or deliver a therapeutically effective amount of a PAI-1 inhibitor and a pharmaceutically acceptable carrier. In some embodiments, a therapeutically effective amount in w/w is about 0.1% to about 5 %, about 0.1 % to about 10%, about 0.1% to about 15% , about 0.1% to about 20%, or about 1% to about 5% . In some embodiments, a therapeutically effective amount is about 10 mg/day to about 100 mg/day, about 10 mg/day to about 200 mg/day, about 10 mg/day to about 300 mg/day, about 10 mg/day to about 400 mg/day, about 10 mg/day to about 500 mg/day, about 10 mg/day to about 600 mg/day, about 10 mg/day to about 700 mg/day, about 10 mg/day to about 800 mg/day, about 10 mg/day to about 900 mg/day, or about 10 mg/day to about 1000 mg/day.

In some embodiments, a provided new composition is characterized in that, when administered to an animal suffering from or susceptible to at least one dermatological condition, it achieves at least one of: (i) treatment of at least one dermatological condition in an animal; (ii) delay, retardation, or prevention of progression of at least one dermatological condition in an animal. In certain particular embodiments, for example when a dermatological condition is or comprises hair graying, a provided new composition may be characterized in that, when administered to an animal suffering from or susceptible to such hair graying, achieves at least one of: (i) a reduction in the number of gray hairs (e.g., present at and/or near a site of administration); (ii) prevention of graying of one or more non-gray hairs (e.g., present at and/or near a site of administration) and (iii) a delayed onset of graying of one or more non-gray hairs (e.g., present at and/or near a site of administration).

In general, administration of a composition in accordance with the present disclosure may be by any of a variety of routes.. In some embodiments, administration is topical. In some embodiments, administration is by injection. In some embodiments, administration is oral.

In some embodiments, administration achieves systemic delivery. In some embodiments, administration achieves local delivery.

In some embodiments administration is or comprises maintaining a composition at or on a site for a period of time. In some embodiments administration is or comprises massaging a composition into a site.

In some embodiments, a composition is maintained at or on a site for a period of time that is at least 1 minute. In some embodiments, a period of time is at least 1 hour. In some embodiments, a period of time is within a range of 1 to 10 minutes. In some embodiments, a period of time is within a range of about 1 to about 10 minutes, about 5 to about 60 minutes, about 5 to about 12 minutes, about 5 to about 15 minutes, or about 15 to about 30 minutes, about 1 to about 12 hours, about 8 to about 12 hours or 12 hours to about 24 hours.

In some embodiments, provided methods of treating a dermatological condition may include, removing administered composition (e.g., removing composition that may remain after a period of time) from its site of administration. In some embodiments, such removing is or comprises rinsing or wiping (e.g., using a wipe that, in some embodiments may be wet or, in some embodiments, may be dry).

In some embodiments, a site to which a composition is administered in accordance with the present disclosure may be on a skin surface. In some embodiments, a site is or comprises hair follicles. In some embodiments a site comprises hair. In some embodiments, a site is or comprises skin overlying a muscle or muscle group. In some embodiments, a site is hairless. In some embodiments, a site is on the torso. In some embodiment a site is on the back. In some embodiments a site is on the chest. In some embodiments, a site is on the buttocks. In some embodiments, a site is on the crotch. In some embodiments, a site is on the groin. In some embodiments, a site is on the head. In some embodiments a site is on the scalp. In some embodiments, a site is on the face. In some embodiments a site is on the neck. In some embodiments a site is on the décolleté. In some embodiments, a site is in the armpit. In some embodiments, a site is on the axillae. In some embodiments a site is on the hands. In some embodiments a site is on the feet. In some embodiments a site is on the arms. In some embodiments a site is on the legs. In some embodiments, a site formerly had hair or hair follicles but no longer has hair or hair follicles. In some embodiments, a site has hair follicles. In some embodiments, hair follicles present at a site have normal structure and/or density. In some embodiments, a site has hair; in some embodiments, such hair is gray but in some embodiments, such hair is not gray.

In some embodiments, a PAI-1 inhibitor for use in accordance with the present disclosure, is or comprises a polypeptide, a nucleic acid, a lipid, a carbohydrate, a small molecule, a metal, or a combination thereof. In some embodiments, a PAI-1 inhibitor is or comprises a polymer (e.g., a polypeptide or polynucleotide). In some embodiments, a PAI-1 inhibitor is or comprises an antibody (e.g., an anti-PAI-1 antibody). In some embodiments, a PAI-1 inhibitor is or comprises a nucleic acid (e.g., is an oligonucleotide, such as an antisense oligonucleotide, an siRNA, etc). In some embodiments, a PAI-1 inhibitor is or comprises a small molecule. In some embodiments, a PAI-1 inhibitors is or comprises 5-Chloro-2-{[(2-{[3-(furan-3-yl)phenyl]amino}-2-oxoethoxy)acetyl]amino benzoic acid, 5-Chloro-2-{[{[3-(furan-3-yl)phenyl]amino}(oxo )acetyl]amino} benzoic acid, a benzopyran compound, a butadiene, spironolactone, imidapril, an angiotensin converting enzyme inhibitor (ACEI, e.g., captopril, or enalapril), an angiotensin II receptor antagonist (AIIRA), a defibrotide (a polydeoxyribonucleotide) or any combination thereof. In some embodiments, a PAI-1 inhibitor is or comprises 5-Chloro-2-{[(2-{[3-(furan-3-yl)phenyl]amino}-2-oxoethoxy)acetyl]amino benzoic acid. According to the claimed invention, the PAI-1 inhibitor is selected from 5-Chloro-2-{[(2-{[3-(furan-3-yl)phenyl]amino}-2-oxoethoxy)acetyl]amino benzoic acid, and 5-Chloro-2-{[{[3-(furan-3-yl)phenyl]amino}(oxo)acetyl]amino} benzoic acid.

In some embodiments, the present disclosure provides and/or utilizes a composition that comprises and/or delivers a PAI-1 inhibitor. In some embodiments, such a composition is or comprises a suspension. In some embodiments, such a composition is or comprises a foam. In some embodiments, such a composition is or comprises an emulsion, e.g., a nanoemulsion. In some embodiments, such a composition is formulated as a suspension, a foam, a lotion, a cream, a gel, an oil, a powder, a liniment, or drops.

In some embodiments, provided methods of treating a dermatological condition comprises a step of administering a penetrating treatment. In some embodiments, a penetrating treatment is or comprises a non-irritating chemical agent. In some embodiments, a penetrating treatment is or comprises administration of an electric or magnetic field. In some embodiments, a penetrating treatment is or comprises microneedling. In some embodiments, a penetrating treatment is or comprises laser treatment.

In some embodiments, provided technologies aide in PAI-1 inhibitor penetration of site of administration. In some embodiments, a provided PAI-1 inhibitor (e.g., in or from a composition as described herein) penetrates its site of administration within about 1, 2, 3, 4, 5, 6, 7, 8, 9, or 10 minutes of administration. In some embodiments, a provided PAI-1 inhibitor penetrates site of administration within about 5 to about 60 minutes, about 5 to about 12 minutes, about 5 to about 15 minutes, or about 15 to about 30 minutes of administration. In some embodiments, a provided PAI-1 inhibitor penetrates site of administration within about 1, 2, 3, 4, 5, 6, 7, 8, 9, 10, 11, 12 or 24 hours of administration. In some embodiments, a provided PAI-1 inhibitor penetrates site of administration within about 1 to about 12 hours, about 8 to about 12 hours or 12 hours to about 24 hours of administration.

In some embodiments, according to the present disclosure, methods of treatment and/or prevention of dermatological conditions comprises two or more administrations of a PAI-1 inhibitor composition over time. In some embodiments, administration of two or more administrations of a composition is separated by a specified period of time. In some embodiments, according to the present disclosure, a specified period of time for administering a composition may be longer than a specified period of time for administering a reference treatment regimen.

Disclosed herein, in certain embodiments, are kits comprising compositions that comprise or deliver a PAI-1 inhibitor.

In some embodiments, provided kits comprise a patch. In some embodiments, a patch is arrange, constructed, and/or utilized for administration to cover an administered composition. Alternatively or additionally, in some embodiments, a patch may contain or comprise a composition that comprises and/or delivers a PAI-1 inhibitor. In some embodiments, a patch comprises microneedles.

In some embodiments, provided kits comprise a device for facilitating penetration of a composition into a site on a subject. In some such embodiments, a provided device may be or comprises a brush, a comb, patch, a roller, a pen, etc.

In some embodiments, provided kits comprise instructions for administering a composition as described herein. In some embodiments, the present disclosure provides insight into administering combination therapy and/or treatment to treat or prevent the occurrence of a dermatological condition. In some embodiments, the combination therapy or treatment, comprises administering a PAI-1 inhibitor as described herein in combination with one or more other active agents. In some embodiments, for example for hair graying, one or more other active agents is selected from the group comprising of cinnamidopropyltrimonium chloride, solid lipid nanoparticles, 1-cystine, 1-methionine, melatonin, and combinations thereof. In some embodiments, for example for keloids, one or more other active agents is selected from the group comprising of pressure therapy, silicone gel sheeting, intra-lesional triamcinolone acetonide (TAC), cryosurgery, radiation, laser therapy, IFN, 5-FU, high doses of oxygen using hyperbaric oxygen therapy (HBOT), cryotherapy, surgical excision, topical agents, and combinations thereof. In some embodiments, for example for scleroderma, one or more other active agents is selected from the group comprising of Angiotensin II Receptor Antagonists, Angiotensin Converting Enzyme (ACE) Inhibitors, NSAIDs, COX-2 Inhibitors, Analgesics, Low-Dose Corticosteroids, Narcotics, Antacids, H2 Blockers, Proton Pump Inhibitors, Prokinetic Agents, Somatostatin Agonist, Antibiotics, Prostaglandin Derivatives, Treprostinil, Iloprost, Endothelin Receptor Antagonists, IP Receptor Agonist, Phosphosdiesterase type 5 (PDE5) inhibitors, Anti-Fibrotic Agent, Tyrosine Kinase Inhibitor, Immunosuppressants, Alkylating agents, Pilocarpine, and combinations thereof. In some embodiments, for example for Raynaud's disease or Raynaud's phenomenon, one or more other active agents is selected from the group comprising of calcium channel blockers, alpha blockers, nitroglycerin, angiotensin II receptor antagonists, selective serotonin reuptake inhibitors, glyceryl trinitrate, tadalafil, Ginkgo biloba extract, SLx-2101, St. John's Wort, fasudil, cilostazol, iloprost, relaxin, treprostinil diethanolamine, sildenafil, atorvastatin, imatinib mesylate, treprostinil diethanolamine, and combinations thereof.

### Definitions

In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the terms "about" and "approximately" may be understood to permit standard variation as would be understood by those of ordinary skill in the art; and (v) where ranges are provided, endpoints are included.

***About:*** The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administration:*** As used herein, the term "administration" typically refers to the administration of a composition to a subject or system to achieve delivery of an agent that is, or is included in, the composition. Those of ordinary skill in the art will be aware of a variety of routes that may, in appropriate circumstances, be utilized for administration to a subject, for example a human. For example, in some embodiments, administration may be ocular, oral, parenteral, topical, etc.. In some particular embodiments, administration may be bronchial (e.g., by bronchial instillation), buccal, dermal (which may be or comprise, for example, one or more of topical to the dermis, intradermal, interdermal, transdermal, etc), enteral, intra-arterial, intradermal, intragastric, intramedullary, intramuscular, intranasal, intraperitoneal, intrathecal, intravenous, intraventricular, within a specific organ (e. g. intrahepatic), mucosal, nasal, oral, rectal, subcutaneous, sublingual, topical, tracheal (e.g., by intratracheal instillation), vaginal, vitreal, etc. In some embodiments, administration may involve only a single dose. In some embodiments, administration may involve application of a fixed number of doses. In some embodiments, administration may involve dosing that is intermittent (e.g., a plurality of doses separated in time) and/or periodic (e.g., individual doses separated by a common period of time) dosing. In some embodiments, administration may involve continuous dosing (e.g., perfusion) for at least a selected period of time.

***Agent** :* In general, the term "agent", as used herein, may be used to refer to a compound or entity of any chemical class including, for example, a polypeptide, nucleic acid, saccharide, lipid, small molecule, metal, or combination or complex thereof. In appropriate circumstances, as will be clear from context to those skilled in the art, the term may be utilized to refer to an entity that is or comprises a cell or organism, or a fraction, extract, or component thereof. Alternatively or additionally, as context will make clear, the term may be used to refer to a natural product in that it is found in and/or is obtained from nature. In some instances, again as will be clear from context, the term may be used to refer to one or more entities that is man-made in that it is designed, engineered, and/or produced through action of the hand of man and/or is not found in nature. In some embodiments, an agent may be utilized in isolated or pure form; in some embodiments, an agent may be utilized in crude form. In some embodiments, potential agents may be provided as collections or libraries, for example that may be screened to identify or characterize active agents within them. In some cases, the term "agent" may refer to a compound or entity that is or comprises a polymer; in some cases, the term may refer to a compound or entity that comprises one or more polymeric moieties. In some embodiments, the term "agent" may refer to a compound or entity that is not a polymer and/or is substantially free of any polymer and/or of one or more particular polymeric moieties. In some embodiments, the term may refer to a compound or entity that lacks or is substantially free of any polymeric moiety.

***Agonist:*** Those skilled in the art will appreciate that the term "agonist" may be used to refer to an agent condition, or event whose presence, level, degree, type, or form correlates with increased level or activity of another agent (i.e., the agonized agent). In general, an agonist may be or include an agent of any chemical class including, for example, small molecules, polypeptides, nucleic acids, carbohydrates, lipids, metals, and/or any other entity that shows the relevant activating activity. In some embodiments, an agonist may be direct (in which case it exerts its influence directly upon its target); in some embodiments, an agonist may be indirect (in which case it exerts its influence by other than binding to its target; e.g., by interacting with a regulator of the target, so that level or activity of the target is altered).

***Antagonist:*** Those skilled in the art will appreciate that the term "antagonist", as used herein, may be used to refer to an agent condition, or event whose presence, level, degree, type, or form correlates with decreased level or activity of another agent (i.e., the inhibited agent, or target). In general, an antagonist may be or include an agent of any chemical class including, for example, small molecules, polypeptides, nucleic acids, carbohydrates, lipids, metals, and/or any other entity that shows the relevant inhibitory activity. In some embodiments, an antagonist may be direct (in which case it exerts its influence directly upon its target); in some embodiments, an antagonist may be indirect (in which case it exerts its influence by other than binding to its target; e.g., by interacting with a regulator of the target, so that level or activity of the target is altered).

***Animal:*** As used herein refers to any member of the animal kingdom. In some embodiments, *"animal"* refers to humans, of either sex and at any stage of development. In some embodiments, *"animal"* refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically engineered animal, and/or a clone.

***Biologically active agent:*** As used herein, the phrase "biologically active agent" refers to any substance that has activity in a biological system and/or organism. For instance, a substance that, when administered to an organism, has a biological effect on that organism is considered to be biologically active. In some embodiments, where a substance (*e.g.,* a polypeptide, nucleic acid, antibody, *etc.*) is biologically active, a portion of that substance that shares at least one biological activity of the whole substance is typically referred to as a "biologically active" portion.

***Carrier:*** as used herein, refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components.

***Cosmetic formulation:*** The term "cosmetic formulation" is used herein to refer to a topically applied composition that contains one or more agents having cosmetic properties. To give but a few examples, a cosmetic formulation may be a skin softener, nutrition lotion type emulsion, cleansing lotion, cleansing cream, skin milk, emollient lotion, massage cream, emollient cream, make-up base, lipstick, facial pack or facial gel, cleaner formulation such as shampoos, rinses, body cleanser, hair-tonics, or soaps, and/or a dermatological composition such as a lotion, ointment, gel, cream, patch, deodorant, antiperspirant, and/or spray.

***Composition:*** Those skilled in the art will appreciate that the term "composition", as used herein, may be used to refer to a discrete physical entity that comprises one or more specified components. In general, unless otherwise specified, a composition may be of any form - e.g., gas, gel, liquid, solid, etc.

***Comprising:*** A composition or method described herein as "comprising" one or more named elements or steps is open-ended, meaning that the named elements or steps are essential, but other elements or steps may be added within the scope of the composition or method. To avoid prolixity, it is also understood that any composition or method described as "comprising" (or which "comprises") one or more named elements or steps also describes the corresponding, more limited composition or method "consisting essentially of" (or which "consists essentially of") the same named elements or steps, meaning that the composition or method includes the named essential elements or steps and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "consists of") the named elements or steps to the exclusion of any other unnamed element or step. In any composition or method disclosed herein, known or disclosed equivalents of any named essential element or step may be substituted for that element or step.

***Cream:*** The term "cream" refers to a spreadable composition, typically formulated for application to the skin. Creams typically contain an oil and/or fatty acid based-matrix. Creams formulated for use in the present invention may contain nanoparticles and may be capable of substantially complete penetration (e.g., of such nanoparticles) through the skin upon topical administration. Such a cream could also act as a carrier for incorporated materials (e.g., for example, for one or more known therapeutic agents and/or independently active biologically active agents).

***Dispersion medium:*** The term "dispersion medium" as used herein, refers to a liquid medium in which particles (*e.g*., empty nanoparticles and/or nanoparticles containing one or more known therapeutic agents and/or independently active biologically active agents) are dispersed. In general, a dispersion is formed when at least two immiscible materials are combined. An "oil-in-water" dispersion is one in which oily particles are dispersed within an aqueous dispersion medium. A "water-in-oil" dispersion is one in which aqueous particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories.

***Dosage form* or *unit dosage form:*** Those skilled in the art will appreciate that the term "dosage form" may be used to refer to a physically discrete unit of an active agent (e.g., a therapeutic or diagnostic agent) for administration to a subject. Typically, each such unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen). Those of ordinary skill in the art appreciate that the total amount of a therapeutic composition or agent administered to a particular subject is determined by one or more attending physicians and may involve administration of multiple dosage forms.

***Dosing regimen:*** Those skilled in the art will appreciate that the term "dosing regimen" may be used to refer to a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which is separated in time from other doses. In some embodiments, individual doses are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount In some embodiments, a dosing regimen is correlated with a desired or beneficial outcome when administered across a relevant population (i.e., is a therapeutic dosing regimen).

***Excipient:*** as used herein, refers to a non-therapeutic agent that may be included in a pharmaceutical composition, for example to provide or contribute to a desired consistency or stabilizing effect. In some embodiments, suitable pharmaceutical excipients may include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***In vitro:*** The term "in vitro" as used herein refers to events that occur in an artificial environment, e.g., in a test tube or reaction vessel, in cell culture, etc., rather than within a multi-cellular organism.

***In vivo:*** as used herein refers to events that occur within a multi-cellular organism, such as a human and a non-human animal. In the context of cell-based systems, the term may be used to refer to events that occur within a living cell (as opposed to, for example, *in vitro* systems).

***Macroemulsion:*** The term "macroemulsion," as used herein, refers to an emulsion in which at least some droplets have diameters in the several hundred nanometers to micrometers size range. As will be understood by those of ordinary skill in the art, a macroemulsion is characterized by droplets greater than 300 nm in diameter. In some embodiments, a macroemulsion composition utilized in accordance with the present disclosure includes one or more large agents or one or more biologically active agents. In some embodiments, a large agent included in a macroemulsion composition may be a biologically active agent. It will be appreciated by those of ordinary skill in the art that a macroemulsion composition for use in accordance with the present disclosure may be prepared according to any available means including, for example, chemical or mechanical means. In some embodiments, droplets in a macroemulsion have a size within a range of about 301 nm and about 1000 µm. In some embodiments, a macroemulsion has droplets in a size distribution of between about 301 nm and about 1000 µm. In some embodiments, droplets in a macroemulsion have a size within a range of about 500 nm and about 5000 µm. In some embodiments, a macroemulsion has droplets in a size distribution of between about 500 nm and about 5000 µm.

***Nanoemulsion:*** The term "nanoemulsion," as used herein, refers to an emulsion in which at least some droplets have diameters in the nanometer size range. As will be understood by those of ordinary skill in the art, a nanoemulsion is characterized by droplets 300 nm or smaller in diameter. In some embodiments, a nanoemulsion composition utilized in accordance with the present disclosure includes one or more large agents or one or more biologically active agents. In some embodiments, a large agent included in a nanoemulsion composition may be a biologically active agent. It will be appreciated by those of ordinary skill in the art that a nanoemulsion composition for use in accordance with the present disclosure may be prepared according to any available means including, for example, chemical or mechanical means. In some embodiments, droplets in a nanoemulsion have a size within a range of about 1 nm and about 300 nm. In some embodiments, a nanoemulsion has droplets in a size distribution of between about 1 nm and about 300 nm.

***Nanoparticle:*** As used herein, the term "nanoparticle" refers to a solid particle having a diameter of less than 300 nm, as defined by the National Science Foundation. In some embodiments, a nanoparticle has a diameter of less than 100 nm as defined by the National Institutes of Health.

***Nanoparticle composition:*** As used herein, the term "nanoparticle composition" refers to any substance that contains at least one nanoparticle. In some embodiments, a nanoparticle composition is a uniform collection of nanoparticles. In some embodiments, nanoparticle compositions are dispersions or emulsions. In general, a dispersion or emulsion is formed when at least two immiscible materials are combined. An "oil-in-water" dispersion is one in which oily particles (or hydrophobic or non-polar) are dispersed within an aqueous dispersion medium. A "water-in-oil" dispersion is one in which aqueous (or hydrophilic or polar) particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to any dispersion medium notwithstanding that it is common to refer to "aqueous" and "oily" categories. In some embodiments, nanoparticle compositions are nanoemulsions. In some embodiments, nanoparticle compositions comprise micelles. In some embodiments, nanoparticle compositions are stable. In some embodiments, nanoparticle compositions include one or more biologically active agents to be delivered in conjunction with the nanoparticles. In some embodiments, nanoparticle compositions are empty nanoparticle compositions (e.g., they do not contain any known therapeutic agents and/or independently active biologically active agents).

***Pharmaceutical composition:*** As used herein, the term "pharmaceutical composition" refers to a composition in which an active agent is formulated together with one or more pharmaceutically acceptable carriers. In some embodiments, the active agent is present in unit dose amount appropriate for administration in a therapeutic regimen that shows a statistically significant probability of achieving a predetermined therapeutic effect when administered to a relevant population. In some embodiments, a pharmaceutical composition may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

***Penetration enhancing agent* or *Penetrating Treatment:*** As used herein, the term "penetration enhancing agent" or "penetrating treatment" refers to an agent whose presence or level correlates with increased penetration of an agent of interest across skin, as compared with that observed in its absence. In some embodiments, a penetration enhancing agent is characterized in that it degrades and/or disrupts skin structure. In some embodiments, a penetration enhancing agent is or comprises a chemical agent (e.g., a chemical or enzyme, for example) For example, chemical agents that that may damage, disrupt, and/or degrade one or more stratum corneum components) may include, for example, alcohols, such as short chain alcohols, long chain alcohols, or polyalcohols; amines and amides, such as urea, amino acids or their esters, amides, AZONE^{®}, derivatives of AZONE^{®}, pyrrolidones, or derivatives of pyrrolidones; terpenes and derivatives of terpenes; fatty acids and their esters; macrocyclic compounds; tensides; or sulfoxides (e.g., dimethylsulfoxide (DMSO), decylmethylsulfoxide, etc.); surfactants, such as anionic, cationic, and nonionic surfactants; polyols; essential oils; and/or hyaluronidase. In some embodiments, a penetration enhancing agent may be an irritant in that an inflammatory and/or allergic reaction occurs when the agent is applied to skin. In some embodiments, a penetration enhancing agent is not an irritant. In some embodiments, a penetration enhancing agent may be or comprise a chemical agent that does not damage, disrupt, or degrade skin structure but whose presence or level nonetheless correlates with increased penetration of an agent of interest across skin, as compared with that observed in its absence. In some embodiments, co-peptides, carrier molecules, and carrier peptides may be penetration enhancing agents which do not damage, disrupt, and/or degrade skin structure(s). In some embodiments, co-peptides, carrier molecules, and carrier peptides may be penetration enhancing agents which do not irritate the skin. The term "penetration enhancing agent" does not encompass mechanical devices (e.g., needles, scalpels, etc.), or equivalents thereof (e.g., other damaging treatments). Also, those skilled in the art will appreciate that a structure such as a nanoparticle or an emulsion is not a chemical agent and therefore not a chemical penetration enhancing agent even if its presence correlates with enhanced skin penetration of an agent of interest that may be associated with the structure.

***Pharmaceutically acceptable carrier:*** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

***Prevent* or *prevention:*** As used herein when used in connection with the occurrence of a disease, disorder, and/or condition, refers to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Self-administration:*** The term "self-administration," as used herein, refers to the situation where a subject has the ability to administer a composition to him or herself without requiring medical supervision. In some embodiments of the invention, self-administration may be performed outside of a clinical setting. To give but one example, in some embodiments of the invention, a facial cosmetic cream may be administered by a subject in one's own home.

***Small molecule:*** As used herein, the term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer. In some embodiments, a small molecule does not include a polymeric moiety. In some embodiments, a small molecule is not and/or does not comprise a protein or polypeptide (e.g., is not an oligopeptide or peptide). In some embodiments, a small molecule is not and/or does not comprise a polynucleotide (e.g., is not an oligonucleotide). In some embodiments, a small molecule is not and/or does not comprise a polysaccharide; for example, in some embodiments, a small molecule is not a glycoprotein, proteoglycan, glycolipid, *etc*.). In some embodiments, a small molecule is not a lipid. In some embodiments, a small molecule is a modulating agent (e.g., is an inhibiting agent or an activating agent). In some embodiments, a small molecule is biologically active. In some embodiments, a small molecule is detectable (e.g., comprises at least one detectable moiety). In some embodiments, a small molecule is a therapeutic agent. Those of ordinary skill in the art, reading the present disclosure, will appreciate that certain small molecule compounds described herein may be provided and/or utilized in any of a variety of forms such as, for example, crystal forms, salt forms, protected forms, pro-drug forms, ester forms, isomeric forms (e.g., optical and/or structural isomers), isotopic forms, etc. Those of skill in the art will appreciate that certain small molecule compounds have structures that can exist in one or more steroisomeric forms. In some embodiments, such a small molecule may be utilized in accoradance with the present disclosure in the form of an individual enantiomer, diastereomer or geometric isomer, or may be in the form of a mixture of stereoisomers; in some embodiments, such a small molecule may be utilized in accordance with the present disclosure in a racemic mixture form. Those of skill in the art will appreciate that certain small molecule compounds have structures that can exist in one or more tautomeric forms. In some embodiments, such a small molecule may be utilized in accoradance with the present disclosure in the form of an individual tautomer, or in a form that interconverts between tautomeric forms. Those of skill in the art will appreciate that certain small molecule compounds have structures that permit isotopic substitution (e.g., ²H or ³H for H;, ¹¹C, ¹³C or ¹⁴C for 12C; , ¹³N or ¹⁵N for 14N; ¹⁷O or ¹⁸O for 16O; ³⁶Cl for XXC; ¹⁸F for XXF; 131I for XXXI; etc). In some embodiments, such a small molecule may be utilized in accordance with the present disclosure in one or more isotopically modified forms, or mixtures thereof. In some embodiments, reference to a particular small molecule compound may relate to a specific form of that compound. In some embodiments, a particular small molecule compound may be provided and/or utilized in a salt form (e.g., in an acid-addition or base-addition salt form, depending on the compound); in some such embodiments, the salt form may be a pharmaceutically acceptable salt form. In some embodiments, where a small molecule compound is one that exists or is found in nature, that compound may be provided and/or utilized in accordance in the present disclosure in a form different from that in which it exists or is found in nature. Those of ordinary skill in the art will appreciate that, in some embodiments, a preparation of a particular small molecule compound that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some embodiments, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc.

***Subject:*** As used herein, the term "subject" refers an organism, typically a mammal (e.g., a human, in some embodiments including prenatal human forms). In some embodiments, a subject is suffering from a relevant disease, disorder or condition. In some embodiments, a subject is susceptible to a disease, disorder, or condition. In some embodiments, a subject displays one or more symptoms or characteristics of a disease, disorder or condition. In some embodiments, a subject does not display any symptom or characteristic of a disease, disorder, or condition. In some embodiments, a subject is someone with one or more features characteristic of susceptibility to or risk of a disease, disorder, or condition. In some embodiments, a subject is a patient. In some embodiments, a subject is an individual to whom diagnosis and/or therapy is and/or has been administered.

***Symptoms are reduced:*** As used herein, the term "symptoms are reduced" refers to when one or more symptoms of a particular disease, disorder or condition is reduced in magnitude (*e.g.,* intensity, severity, *etc.*) and/or frequency. For purposes of clarity, a delay in the onset of a particular symptom is considered one form of reducing the frequency of that symptom.

***Therapeutic agent:*** As used herein, the term "therapeutic agent" refers to any agent that has a therapeutic effect and/or elicits a desired biological and/or pharmacological effect, when administered to a subject.

***Therapeutically effective amount:*** As used herein, is meant an amount that produces the desired effect for which it is administered. In some embodiments, the term refers to an amount that is sufficient, when administered to a population suffering from or susceptible to a disease, disorder, and/or condition in accordance with a therapeutic dosing regimen, to treat the disease, disorder, and/or condition. In some embodiments, a therapeutically effective amount is one that reduces the incidence and/or severity of, and/or delays onset of, one or more symptoms of the disease, disorder, and/or condition. Those of ordinary skill in the art will appreciate that the term *"therapeutically effective amount"* does not in fact require successful treatment be achieved in a particular individual. Rather, a therapeutically effective amount may be that amount that provides a particular desired pharmacological response in a significant number of subjects when administered to patients in need of such treatment. In some embodiments, reference to a therapeutically effective amount may be a reference to an amount as measured in one or more specific tissues (e.g., a tissue affected by the disease, disorder or condition) or fluids (e.g., blood, saliva, serum, sweat, tears, urine, etc.). Those of ordinary skill in the art will appreciate that, in some embodiments, a therapeutically effective amount of a particular agent or therapy may be formulated and/or administered in a single dose. In some embodiments, a therapeutically effective agent may be formulated and/or administered in a plurality of doses, for example, as part of a dosing regimen.

***Treatment:*** As used herein, the term "treatment" (also "treat" or "treating") refers to any administration of a therapy that partially or completely alleviates, ameliorates, relives, inhibits, delays onset of, reduces severity of, and/or reduces incidence of one or more symptoms, features, and/or causes of a particular disease, disorder, and/or condition. In some embodiments, such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

### Detailed Description of Certain Embodiments

### Plasminogen Activator Inhibitor 1 (PAI-1)

Plasminogen Activator Inhibitor (PAI-1) is a serine protease inhibitor (serpin) protein encoded by the SERPINE 1 gene. PAI-1 is originally known for its involvement in maintaining homeostatic equilibrium in the body, as it is the principal inhibitor of tissue plasminogen activator (tPA) and urokinase (uPA). Elevated PAI-1 has also been reported to be associated with organ fibrosis and disease in multiple organ systems (e.g., heart, lung, liver, kidney, and skin).

The present disclosure, in some embodiments, encompasses the recognition that PAI-1 inhibitors may be particularly useful in the treatment and/or prevention of dermatological conditions, such as hair graying, scleroderma, keloids, etc.. While the growth cycle and physiology of the hair is well known and understood, there are currently no effective prevention or treatment techniques for hair graying. In some embodiments, provided methods and/or compositions provide targeted therapy. For example, in some embodiments, provided methods and compositions provide surprisingly effective therapies comprising one or more PAI-1 inhibitors. Without wishing to be bound by any particular theory, it is proposed that, in some embodiments, administration of a PAI-1 inhibitor as described herein may stimulate Hair Follicle Stem Cells (HFSC), and such stimulation may contribute to treatment or prevention of hair graying.

In some embodiments provided methods, kits and compositions may be or comprise emulsions. In some embodiments provided methods, kits and compositions may be or comprise macroemulsions. In some embodiments provided methods, kits and compositions may be or comprise nanoemulsions. In some embodiments provided methods, kits and compositions comprise a combination therapy or treatment, wherein for example, in some embodiments, provided compositions may be administered in combination with one or more additional treatments. In some embodiments the one or more additional treatments is or comprises other active agents and/or therapeutic modalities (e.g. one or more PAI-inhibitors, or other agents), such as known therapeutic agents and/or independently active biologically active agents.

### Diseases, Disorders, and Conditions

The present disclosure provides technologies for treating and/or preventing certain dermatologic diseases, disorders, and/or conditions. In some embodiments, the present disclosure provides technologies for treating and/or preventing diseases, disorders or conditions associated with the epidermal and/or dermal level of the skin.

In some embodiments, the present disclosure provides technologies for treating and/or preventing one or more of hair graying, keloids, scleroderma, Raynaud's Disease (or Raynaud's Phenomenon), and/or combinations thereof. In some embodiments, the present disclosure provides technologies for treating and/or preventing hair graying.

### Hair Graying

In some embodiments, the present disclosure provides technologies for treating and/or preventing a dermatological condition. In some embodiments, the present disclosure provides technologies for treating and/or preventing hair graying.

Current therapies for hair graying include, but is not limited to photoprotectors, such as cinnamidopropyltrimonium chloride and solid lipid nanoparticles as carriers for UV blockers, oral supplementation with 1-cystine and 1-methionine, and topical melatonin. The most commonly used treatment or solution to premature hair graying is the use of temporary hair colorants. Pharmaceutical compositions in accordance with the present disclosure may be administered alone and/or in combination with these therapies that are used to treat the symptoms and/or causes of hair graying, for the treatment of hair graying.

In some embodiments, provided compositions for treatment and/or prevention of hair graying are formulated into a suspension.

In some embodiments, the compositions disclosed herein for treatment and/or prevention of hair graying are formulated into a suspension, a foam, a cream, a liniment, a lotion, a gel, a shampoo, a conditioner, etc.

In some embodiments, compositions disclosed herein for treatment and/or prevention of hair graying are administered locally to an affected site (e.g., scalp, hair follicle, face, neck, back, arms, chest, etc.).

Administration of the disclosed compositions and/or formulations may be through any one of many routes. In some embodiments, the administration is topical. In some embodiments, the administration is oral. In some embodiments, the administration is via an injection.

In some embodiments, administration achieves systemic delivery. In some embodiments, administration achieves local delivery.

In some embodiments, the present disclosure involves administration of at least one therapeutic agent (*e.g.,* PAI-1 inhibitor) in a suspension, in an amount sufficient to achieve a reduction in the degree and/or prevalence of one or more symptoms of hair graying of at least about 25%; in some embodiments in an amount sufficient to achieve a reduction in the degree and/or prevalence of one or more symptoms of hair graying of at least about 30%; in some embodiments in an amount sufficient to achieve a reduction in the degree and/or prevalence of one or more symptoms of hair graying of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

In some embodiments, the present disclosure involves administration of at least one therapeutic agent (*e.g.,* PAI-1 inhibitor) in a nanoparticle composition or a nanoemulsion composition or an emulsion composition or a foam formulation or a cream formulation or an oil or a lotion formulation or a gel or a shampoo or a conditioner, in an amount sufficient to achieve a reduction in the degree and/or prevalence of one or more symptoms of hair graying of at least about 25%; in some embodiments in an amount sufficient to achieve a reduction in the degree and/or prevalence of one or more symptoms of hair graying of at least about 30%; in some embodiments in an amount sufficient to achieve a reduction in the degree and/or prevalence of one or more symptoms of hair graying of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Keloids (not part of the invention)

Keloids are proliferated scar tissue that forms on the site of a cutaneous injury. In a particular example, the site of a cutaneous injury is a surgical incision or a site of trauma. Unlike hypertrophic scars, which are raised scars, keloids grow beyond the boundaries of the original wound. Keloids result from an overgrowth of granulation tissue or collagen type 3 at the site of the cutaneous injury. Keloids may be firm,rubbery lesions. In some embodiments, keloids may be shiny, fibrous nodules. While these are benign growth and not contagious, keloids may sometimes be accompanied with pain, itchiness, and may affect movement of the skin. An estimated 15% of African Americans and Hispanics have keloids.

While there is no effective treatment, current therapies for keloids include pressure therapy, silicone gel sheeting, intra-lesional triamcinolone acetonide (TAC), cryosurgery, radiation, laser therapy, IFN, 5-FU, high doses of oxygen using hyperbaric oxygen therapy (HBOT), cryotherapy, and surgical excision as well as a multitude of extracts and topical agents. The most common form of treatment is currently surgical excision. However, the best treatment is prevention in patients with known predisposition. Pharmaceutical compositions in accordance with the present disclosure may be administered alone and/or in combination with these therapies that are used to treat the symptoms and/or causes of keloids, for the treatment of keloids.

In some embodiments, provided compositions for treatment and/or prevention of keloids are formulated into a suspension.

In some embodiments, provided compositions for treatment and/or prevention of keloids are formulated into a suspension, a foam, a cream, a liniment, a lotion, a gel, a shampoo, a conditioner, etc.

In some embodiments, provided compositions for treatment and/or prevention of keloids are administered locally to an affected site (e.g., scalp, hair follicle, face, neck, back, arms, chest, etc.).

Administration of the disclosed compositions and/or formulations may be through any one of many routes. In some embodiments, the administration is topical. In some embodiments, the administration is oral. In some embodiments, the administration is via an injection.

In some embodiments, administration achieves systemic delivery. In some embodiments, administration achieves local delivery.

In some embodiments, the present disclosure involves administration of at least one therapeutic agent (*e.g.,* PAI-1 inhibitor) in a suspension, in an amount sufficient to achieve a reduction in the degree and/or prevalence of one or more symptoms of keloids of at least about 25%; in some embodiments in an amount sufficient to achieve a reduction in the degree and/or prevalence of one or more symptoms of keloids of at least about 30%; in some embodiments in an amount sufficient to achieve a reduction in the degree and/or prevalence of one or more symptoms of keloids of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

In some embodiments, the present disclosure involves administration of at least one therapeutic agent (*e.g.,* PAI-1 inhibitor) in a nanoparticle composition or a nanoemulsion composition or an emulsion composition or a foam formulation or a cream formulation or an oil or a lotion formulation or a gel or a shampoo or a conditioner, in an amount sufficient to achieve a reduction in the degree and/or prevalence of one or more symptoms of keloids of at least about 25%; in some embodiments in an amount sufficient to achieve a reduction in the degree and/or prevalence of one or more symptoms of keloids of at least about 30%; in some embodiments in an amount sufficient to achieve a reduction in the degree and/or prevalence of one or more symptoms of keloids of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Scleroderma (not part of the invention)

Scleroderma, or systemic sclerosis, is generally considered a chronic systemic autoimmune disease characterized, among other things, fibrosis or hardening, vascular alterations, and autoantibodies.

In some embodiments, scleroderma is also considered a connective tissue disease generally characterized with an excessive accumulation of Extracellular Matrix proteins in the skin and internal organs, vascular injury, and immunological abnormalities.

Many of the clinical manifestations of the disease are thought to involve a misregulation of vascular remodeling. One of the earliest symptoms of scleroderma is microvascular injury. This microvascular injury is thought to cause increased endothelial cell activation. Activated endothelial cells are believed to express adhesion molecules resulting in altered capillary permeability allowing migration of inflammatory cells through the endothelium and entrapment in the vessel wall. The immune activation is thought to contribute to sustained endothelial activation, which results in the breakdown of endothelial cells. This process is believed to contribute to the loss of elasticity and narrowing of the vessels commonly observed in scleroderma patients. Furthermore, it is thought that microvascular injury contributes to perivascular infiltrates of mononuclear cells in the dermis which is thought to contribute to the activation of fibroblasts and many of the associated hallmark symptoms of scleroderma. As fibrosis increases, permeability decreases. As a result, it becomes more difficult for antibodies to penetrate diseased tissues. Therefore, the affinity of anti-CCL2 antibodies becomes particularly important to keep antibodies localized.

Many of the clinical manifestations of the disease are generally thought to involve the misregulation of fibroblasts. The main function of fibroblasts is to maintain the structural integrity of connective tissues by continuously secreting precursors of the extracellular matrix. Fibroblasts provide a structural framework (stroma) for many tissues, play an important role in wound healing and are the most common cells of connective tissue in animals. Fibroblasts are morphologically heterogeneous with diverse appearances depending on their location and activity.

There are two major forms of scleroderma: limited systemic sclerosis/scleroderma and diffuse systemic sclerosis/scleroderma. In limited cutaneous scleroderma, the fibrosis of the skin is generally confined to the area. Patients with limited cutaneous scleroderma generally experience vascular impairment. Cutaneous and organ fibrosis generally progresses slowly in patients with limited scleroderma. Patients with diffuse scleroderma generally experience fibrosis of skin and organs that progresses more rapidly than in limited scleroderma and/or widespread inflammation and/or more severe internal organ involvement than is seen in limited scleroderma.

It is generally thought that interstitial lung disease, resulting in pulmonary fibrosis, is the leading cause of scleroderma related deaths (Ludwicka-Bradley, A., et al. Coagulation and autoimmunity in scleroderma interstitial lung disease. Semin Arthritis Rheum, 41(2), 212-22, 2011). Further complications resulting in scleroderma-related deaths include but are not limited to cancer, heart failure, pulmonary hypertension, kidney failure, and malabsorption, or any combination thereof.

Scleroderma is most commonly diagnosed by inspection of skin symptoms. Tests to diagnosis include but are not limited to visual and/or manual inspection of the skin, blood pressure testing, chest x-ray, lung CT, echocardiogram, urinalysis, skin biopsy, and blood tests including antinuclear antibody testing, anti-topoisomerase antibody testing, anti-centromere antibody testing, anti-U3 antibody testing, anti-RNA antibody testing, other types of antibody testing, erythrocyte sedimentation rate, and rheumatoid factor. There is currently no known cure for scleroderma. Treatments administered are designed to treat the symptoms of the disease. Treatments include Angiotensin II Receptor Antagonists, Angiotensin Converting Enzyme (ACE) Inhibitors, NSAIDs, COX-2 Inhibitors, Analgesics, Low-Dose Corticosteroids, Narcotics, Antacids, H2 Blockers, Proton Pump Inhibitors, Prokinetic Agents, Somatostatin Agonist, Antibiotics, Prostaglandin Derivatives, Treprostinil, Iloprost, Endothelin Receptor Antagonists, IP Receptor Agonist, Phosphosdiesterase type 5 (PDE5) inhibitors, Anti-Fibrotic Agent, Tyrosine Kinase Inhibitor, Immunosuppressants, Alkylating agents, Pilocarpine, and combinations thereof.

In some embodiments, provided compositions for treatment and/or prevention of scleroderma are formulated into a suspension.

In some embodiments, provided compositions for treatment and/or prevention of scleroderma are formulated into a suspension, a foam, a cream, a liniment, a lotion, a gel, a shampoo, a conditioner, etc.

In some embodiments, provided compositions for treatment and/or prevention of scleroderma are administered locally to an affected site (e.g., scalp, hair follicle, face, neck, back, arms, chest, etc.).

Administration of the disclosed compositions and/or formulations may be through any one of many routes. In some embodiments, the administration is topical. In some embodiments, the administration is oral. In some embodiments, the administration is via an injection.

In some embodiments, administration achieves systemic delivery. In some embodiments, administration achieves local delivery.

In some embodiments, the present disclosure involves administration of at least one therapeutic agent (*e.g.,* PAI-1 inhibitor) in a suspension, in an amount sufficient to achieve a reduction in the degree and/or prevalence of one or more symptoms of scleroderma of at least about 25%; in some embodiments in an amount sufficient to achieve a reduction in the degree and/or prevalence of one or more symptoms of scleroderma of at least about 30%; in some embodiments in an amount sufficient to achieve a reduction in the degree and/or prevalence of one or more symptoms of scleroderma of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

In some embodiments, the present disclosure involves administration of at least one therapeutic agent (*e.g.,* PAI-1 inhibitor) in a nanoparticle composition or a nanoemulsion composition or an emulsion composition or a foam formulation or a cream formulation or an oil or a lotion formulation or a gel or a shampoo or a conditioner, in an amount sufficient to achieve a reduction in the degree and/or prevalence of one or more symptoms of scleroderma of at least about 25%; in some embodiments in an amount sufficient to achieve a reduction in the degree and/or prevalence of one or more symptoms of scleroderma of at least about 30%; in some embodiments in an amount sufficient to achieve a reduction in the degree and/or prevalence of one or more symptoms of scleroderma of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Raynaud's Phenomenon (not part of the invention)

Raynaud's phenomenon or Raynaud's disease is a vasospastic condition of the fingers and toes. Typically in response to cold or emotional stress, the skin of the fingers become discolored (white, blue, and/or red, often in this sequence) and painful. Severe Raynaud's can result in necrosis of the skin and ultimately the fingers and/or toes, resulting in "auto-amputation." Nails of Raynaud's patients may become brittle. This condition is frequently associated with connective tissue diseases such as scleroderma and/or rheumatoid arthritis.

Pharmaceutical compositions in accordance with the present disclosure may be administered alone and/or in combination with other agents that are used to treat the symptoms and/or causes of Raynaud's phenomenon, for the treatment of Raynaud's phenomenon. In some embodiments, such agents include calcium channel blockers (*e.g.*, nifedipine, *etc.*)*,* alpha blockers (*e.g.,* hydralazine, *etc.*)*,* nitroglycerin, angiotensin II receptor antagonists (*e.g.,* losartan, *etc.*)*,* selective serotonin reuptake inhibitors (*e.g.,* fluoxetine, *etc.*)*,* glyceryl trinitrate, tadalafil, *Ginkgo biloba* extract, SLx-2101, St. John's Wort, fasudil, cilostazol, iloprost, relaxin, treprostinil diethanolamine, sildenafil, atorvastatin, imatinib mesylate, treprostinil diethanolamine, and/or combinations thereof.

In some embodiments, provided compositions for treatment and/or prevention of Raynaud's disease are formulated into a suspension.

In some embodiments, the compositions disclosed herein for treatment and/or prevention of Raynaud's disease are formulated into a suspension, a foam, a cream, a liniment, a lotion, a gel, a shampoo, a conditioner, etc.

In some embodiments, compositions disclosed herein for treatment and/or prevention of Raynaud's disease are administered locally to an affected site (e.g., nose, lips, ears, nipples, fingers, toes, scalp, face, neck, back, arms, chest, etc.).

Administration of the disclosed compositions and/or formulations may be through any one of many routes. In some embodiments, the administration is topical. In some embodiments, the administration is oral. In some embodiments, the administration is via an injection.

In some embodiments, administration achieves systemic delivery. In some embodiments, administration achieves local delivery.

In some embodiments, the present disclosure involves administration of at least one therapeutic agent (*e.g.,* PAI-1 inhibitor) in a suspension, in an amount sufficient to achieve a reduction in the degree and/or prevalence of one or more symptoms of Raynaud's phenomenon of at least about 25%; in some embodiments in an amount sufficient to achieve a reduction in the degree and/or prevalence of one or more symptoms of Raynaud's phenomenon of at least about 30%; in some embodiments in an amount sufficient to achieve a reduction in the degree and/or prevalence of one or more symptoms of Raynaud's phenomenon of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

In some embodiments, the present disclosure involves administration of at least one therapeutic agent (*e.g.,* PAI-1 inhibitor) in a nanoparticle composition or a nanoemulsion composition or an emulsion composition or a foam formulation or a cream formulation or an oil or a lotion formulation or a gel or a shampoo or a conditioner, in an amount sufficient to achieve a reduction in the degree and/or prevalence of one or more symptoms of Raynaud's phenomenon of at least about 25%; in some embodiments in an amount sufficient to achieve a reduction in the degree and/or prevalence of one or more symptoms of Raynaud's phenomenon of at least about 30%; in some embodiments in an amount sufficient to achieve a reduction in the degree and/or prevalence of one or more symptoms of Raynaud's phenomenon of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### PAI-1 Inhibitors

PAI-1 inhibitors can be used to treat or prevent medical conditions or diseases associated with over-expression of PAI-1. PAI-1 inhibitors can be antibodies, peptides, polypeptides, proteins, nucleic acids, lipids, carbohydrates, small molecules, metals, polymers, therapeutic antibodies, or any combinations thereof. In some embodiments, the PAI-1 inhibitor is an siRNA. In some embodiments, the PAI-1 inhibitor is a benzopyran compound, a butadiene, spironolactone, imidapril, an angiotensin converting enzyme inhibitor (ACEI, captopril, or enalapril), an angiotensin II receptor antagonist (AIIRA), a defibrotide (a polydeoxyribonucleotide) and any combination thereof. In some embodiments, the PAI-1 inhibitor is a benzopyran compound.

In some embodiments, the PAI-1 inhibitor may be a small molecule. For example, the PAI-1 inhibitor is 5-Chloro-2-{[(2-{[3-(furan-3-yl)phenyl]amino}-2-oxoethoxy)acetyl]amino benzoic acid. In another example, the PAI-1 inhibitor is 5-Chloro-2-{[{[3-(furan-3-yl)phenyl]amino}(oxo )acetyl]amino }benzoic acid. Table 1 below lists the exemplary PAI-1 inhibitors. According to the claimed invention, the PAI-1 inhibitor is selected from 5-Chloro-2-{[(2-{[3-(furan-3-yl)phenyl]amino}-2-oxoethoxy)acetyl] amino benzoic acid, and 5-Chloro-2-{[{[3-(furan-3-yl)phenyl]amino}(oxo)acetyl]amino} benzoic acid.

**Table Exemplary PAI-1 inhibitors**

| **Chemical Name** | **Molecular Formula** | **Molecular Weight** |
|---|---|---|
| 5-Chloro-2-{[(2-{[3-(furan-3-yl)phenyl]amino}-2-oxoethoxy)acetyl] amino benzoic acid | C₂₁H₁₇ClN₂O₆ | 428.82 |
| 5-Chloro-2-{ [{ [3-(furan-3-yl)phenyl]amino}(oxo )acetyl]amino} benzoic acid | C₁₉H₁₃ClN₂O₅ | 384.77 |

### Compositions and Formulations

As noted herein, the present invention utilizes compositions comprising one or more PAI-1 inhibitors for administration. In some embodiments the administration is in combination with microneedle skin conditioning (MSC). In some embodiments, provided compositions may be formulated for topical and/or transdermal delivery (e.g., as lotions, creams, liniments, ointments, powders, gels, drops, etc.). In some embodiments, provided compositions may be or include a nanoemulsion. In some embodiments, provided compositions may be or include a macroemulsion.

Nanoparticle compositions are useful in a variety of contexts, and have proven to be particularly useful and/or effective in the context of medical applications, including administering therapeutic agents (e.g., PAI-1 inhibitors) to patients in need thereof. Nanoparticle compositions have proven to be particularly useful and/or effective in the context of topical administration of therapeutic agents (see, *e.g.,* PCT patent application number PCT US06/46236, filed December 1, 2006, published as WO 08/045107 on April 17, 2008, and entitled "BOTULINUM NANOEMULSIONS; in PCT patent application number PCT US07/86018, filed November 30, 2007, published as WO 08/070538 on June 12, 2008, and entitled "AMPHIPHILIC ENTITY NANOPARTICLES"; and/or in PCT patent application number PCT US09/48972, filed June 26, 2009, published as WO 09/158687 on December 30, 2009, and entitled "DERMAL DELIVERY").

In some embodiments, provided nanoparticle compositions have particular components, and/or relative amounts of components, as described herein. In some embodiments, provided nanoparticle compositions have particular structural and/or functional attributes that distinguish and/or define them. In some embodiments, exemplary attributes (*e.g.*, physical, structural, and/or functional attributes) that have been associated with nanoparticle compositions in general are described in the following paragraphs. In some embodiments, provided nanoparticle compositions have one or more of these attributes. In some embodiments, provided nanoparticle compositions do not have any of these attributes.

In general, a nanoparticle composition is any composition that includes at least one nanoparticle. In some embodiments, nanoparticle compositions comprise at least one known therapeutic agent and/or an independently active biologically active agent (*e.g.,* PAI-1 inhibitor). A known therapeutic agent and/or an independently active biologically active agent may be encapsulated or completely surrounded by one or more nanoparticles; associated with the nanoparticle interface; and/or adsorbed to the outer surface of one or more nanoparticles. A known therapeutic agent and/or an independently active biologically active agent may or may not be covalently linked to the nanoparticles and/or nanoparticle compositions; a known therapeutic agent and/or an independently active biologically active agent may or may not be attached to nanoparticles and/or nanoparticle compositions by adsorption forces. In some embodiments, nanoparticle compositions comprise empty nanoparticles (*e.g*., nanoparticles not containing any known therapeutic agents and/or independently active biologically active agents).

In some embodiments, nanoparticle compositions are stable. In some embodiments, nanoparticle compositions are uniform. For example, in some embodiments, the difference between the minimum diameter and maximum diameter of the nanoparticles in a nanoparticle composition does not exceed approximately 600 nm, approximately 550 nm, approximately 500 nm, approximately 450 nm, approximately 400 nm, approximately 350 nm, approximately 300 nm, approximately 250 nm, approximately 200 nm, approximately 150 nm, or approximately 100 nm, approximately 90 nm, approximately 80 nm, approximately 70 nm, approximately 60 nm, approximately 50 nm, or fewer nm.

In some embodiments, particles within nanoparticle compositions have diameters (*e.g.,* average and/or median diameters) that are smaller than about 1000 nm, about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, about 50 nm, or less.

In some embodiments, particles within nanoparticle compositions have diameters (*e.g*., average and/or median diameters) within the range of about 10 nm and about 600 nm. In some embodiments, particles within nanoparticle compositions have diameters (*e.g.,* average and/or median diameters) within the range of about 10 nm to about 300 nm, about 10 nm to about 200 nm, about 10 nm to about 150 nm, about 10 nm to about 130 nm, about 10 nm to about 120 nm, about 10 nm to about 115 nm, about 10 nm to about 110 nm, about 10 nm to about 100 nm, or about 10 nm to about 90 nm. In some embodiments, particles within nanoparticle compositions have diameters (*e.g.,* average and/or median diameters) within the range of 1 nm to 1000 nm, 1 nm to 600 nm, 1 nm to 500 nm, 1 nm to 400 nm, 1 nm to 300 nm, 1 nm to 200 nm, 1 nm to 150 nm, 1 nm to 120 nm, 1 nm to 100 nm, 1 nm to 75 nm, 1 nm to 50 nm, or 1 nm to 25 nm. In some embodiments, particles within nanoparticle compositions have diameters (*e.g.,* average and/or median diameters) of 1 nm to 15 nm, 15 nm to 200 nm, 25 nm to 200 nm, 50 nm to 200 nm, or 75 nm to 200 nm.

In some embodiments, the total particle distribution is encompassed within the specified range of particle diameter size. In some embodiments, less than 50%, 25%, 10%, 5%, or 1% of the total particle distribution is outside of the specified range of particle diameter sizes. In some embodiments, less than 1% of the total particle distribution is outside of the specified range of particle diameter sizes. In certain embodiments, the nanoparticle composition is substantially free of particles having a diameter larger than 300 nm, 250 nm, 200 nm, 150 nm, 120 nm, 100 nm, 75 nm, 50 nm, or 25 nm. In some embodiments, less than 50%, 25%, 10%, 5%, or 1% of the total particle distribution have diameters larger than 300 nm, 250 nm, 200 nm, 150 nm, 120 nm, 100 nm, 75 nm, 50 nm, or 25 nm.

In some embodiments, particles within nanoparticle compositions have an average particle size that is under about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, or about 50 nm. In some embodiments, the average particle size is within the range of about 10 nm and about 300 nm, about 50 nm and about 250, about 60 nm and about 200 nm, about 65 nm and about 150 nm, or about 70 nm and about 130 nm. In some embodiments, the average particle size is about 80 nm and about 110 nm. In some embodiments, the average particle size is about 90 nm and about 100 nm.

In some embodiments, a majority of the particles within nanoparticle compositions have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition.

In some embodiments, nanoparticle compositions are substantially free of particles having a diameter in excess of 600 nm. Specifically, in some embodiments, fewer than 50% of the nanoparticles in nanoparticle compositions have a diameter in excess of 600 nm. In some embodiments, fewer than 25% of the particles have a diameter in excess of 600 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 600 nm. Furthermore, in some embodiments, the nanoparticles in nanoparticle compositions have diameters within the range of 10 nm and 600 nm.

In some embodiments, nanoparticle compositions are substantially free of particles having a diameter in excess of 500 nm. Specifically, in some embodiments, fewer than 50% of the nanoparticles in nanoparticle compositions have a diameter in excess of 500 nm. In some embodiments, fewer than 25% of the particles have a diameter in excess of 500 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 500 nm. Furthermore, in some embodiments, the nanoparticles in nanoparticle compositions have diameters within the range of 10 nm and 500 nm.

In some embodiments, nanoparticle compositions are substantially free of particles having a diameter in excess of 400 nm. Specifically, in some embodiments, fewer than 50% of the nanoparticles in nanoparticle compositions have a diameter in excess of 400 nm. In some embodiments, fewer than 25% of the particles have a diameter in excess of 400 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 400 nm. Furthermore, in some embodiments, the nanoparticles in nanoparticle compositions have diameters within the range of 10 nm and 400 nm.

In some embodiments, nanoparticle compositions are substantially free of particles having a diameter in excess of 300 nm. Specifically, in some embodiments, fewer than 50%, of the nanoparticles in nanoparticle compositions have a diameter in excess of 300 nm. In some embodiments, fewer than 25% of the particles have a diameter in excess of 300 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 300 nm. Furthermore, in some embodiments, the nanoparticles in nanoparticle compositions have diameters within the range of 10 nm and 300 nm.

In some embodiments, nanoparticle compositions are substantially free of particles having a diameter in excess of 200 nm. Specifically, in some embodiments, fewer than 50%, of the nanoparticles in nanoparticle compositions have a diameter in excess of 200 nm. In some embodiments, fewer than 25% of the particles have a diameter in excess of 200 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 200 nm. Furthermore, in some embodiments, the nanoparticles in nanoparticle compositions have diameters within the range of 10 nm and 200 nm.

In some embodiments, provided compositions are substantially free of particles having a diameter in excess of 150 nm. Specifically, in some embodiments, fewer than 50% of the nanoparticles in provided compositions have a diameter in excess of 150 nm. In some embodiments, fewer than 25% of the particles have a diameter in excess of 150 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 150 nm. Furthermore, in some embodiments, the nanoparticles in provided compositions have diameters within the range of 10 nm and 150 nm.

In some embodiments, nanoparticle compositions are substantially free of particles having a diameter in excess of 120 nm. Specifically, in some embodiments, fewer than 50%, of the nanoparticles in nanoparticle compositions have a diameter in excess of 120 nm. In some embodiments, fewer than 25% of the particles have a diameter in excess of 120 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 120 nm. Furthermore, in some embodiments, the nanoparticles in nanoparticle compositions have diameters within the range of 10 nm and 120 nm.

In some embodiments, a majority of particles in a provided composition have diameters (*e.g.,* average and/or median diameters) between 10 nm and 150 nm. In some embodiments, a majority of particles in a provided composition have diameters (*e.g.,* average and/or median diameters) between 10 nm and 120 nm. In some embodiments, a majority of particles in a provided composition have diameters (*e.g.,* average and/or median diameters) between 20 nm and 120 nm. In some embodiments, a majority of particles in a provided composition have diameters (*e.g.,* average and/or median diameters) between 20 nm and 110 nm. In some embodiments, a majority of particles in a provided composition have diameters (*e.g.,* average and/or median diameters) between 20 nm and 100 nm. In some embodiments, a majority of particles in a provided composition have diameters (*e.g.,* average and/or median diameters) between 20 nm and 90 nm. In some embodiments, a majority of particles in a provided composition have diameters (*e.g.,* average and/or median diameters) between 20 nm and 80 nm. In some embodiments, a majority of particles in a provided composition have diameters (*e.g.,* average and/or median diameters) between 20 nm and 70 nm. In some embodiments, a majority of particles in a provided composition have diameters (*e.g.,* average and/or median diameters) between 20 nm and 60 nm. In some embodiments, a majority of particles in a provided composition have diameters (*e.g.,* average and/or median diameters) between 20 nm and 50 nm. In some embodiments, a majority of particles in a provided composition have diameters (*e.g.,* average and/or median diameters) between 20 nm and 40 nm. In some embodiments, a majority of particles in a provided composition have diameters (*e.g.,* average and/or median diameters) between 20 nm and 30 nm.

In some embodiments, a majority of nanoparticles in a nanoparticle composition have diameters (*e.g.,* average and/or median diameters) between 10 nm and 120 nm. In some embodiments, a majority of nanoparticles in a nanoparticle composition have diameters (*e.g.,* average and/or median diameters) between 20 nm and 120 nm. In some embodiments, a majority of nanoparticles in a nanoparticle composition have diameters (*e.g.,* average and/or median diameters) between 20 nm and 110 nm. In some embodiments, a majority of nanoparticles in a nanoparticle composition have diameters (*e.g.,* average and/or median diameters) between 20 nm and 100 nm. In some embodiments, a majority of nanoparticles in a nanoparticle composition have diameters between 20 nm and 90 nm. In some embodiments, a majority of nanoparticles in a nanoparticle composition have diameters (*e.g.,* average and/or median diameters) between 20 nm and 80 nm. In some embodiments, a majority of nanoparticles in a nanoparticle composition have diameters (*e.g.,* average and/or median diameters) between 20 nm and 70 nm. In some embodiments, a majority of nanoparticles in a nanoparticle composition have diameters (*e.g.,* average and/or median diameters) between 20 nm and 60 nm. In some embodiments, a majority of nanoparticles in a nanoparticle composition have diameters (*e.g.,* average and/or median diameters) between 20 nm and 50 nm. In some embodiments, a majority of nanoparticles in a nanoparticle composition have diameters (*e.g.,* average and/or median diameters) between 20 nm and 40 nm. In some embodiments, a majority of nanoparticles in a nanoparticle composition have diameters (*e.g.,* average and/or median diameters) between 20 nm and 30 nm.

In some embodiments, about 50% of nanoparticles in a nanoparticle composition have diameters (*e.g.,* average and/or median diameters) between 10 nm and 40 nm. In some embodiments, about 90% of nanoparticles in a nanoparticle composition have diameters (*e.g.,* average and/or median diameters) between 10 nm and 80 nm. In some embodiments, about 90% of nanoparticles in a nanoparticle composition have diameters (*e.g.,* average and/or median diameters) between 10 nm and 90 nm. In some embodiments, about 95% of nanoparticles in a nanoparticle composition have diameters (*e.g.,* average and/or median diameters) between 10 nm and 110 nm. In some embodiments, about 95% of nanoparticles in a nanoparticle composition have diameters (*e.g.,* average and/or median diameters) between 10 nm and 120 nm. In some embodiments, about 95% of particles in a provided composition have diameters (*e.g.,* average and/or median diameters) between 10 nm and 150 nm.

In some embodiments, about 50% of the aggregate volume of all nanoparticles in a nanoparticle composition comprises or consists of nanoparticles having diameters between 10 nm and 40 nm. In some embodiments, about 90% of the aggregate volume of all nanoparticles in a nanoparticle composition comprises or consists of nanoparticles having diameters between 10 nm and 80 nm. In some embodiments, about 95% of the aggregate volume of all nanoparticles in a nanoparticle composition comprises or consists of nanoparticles having diameters between 10 nm and 110 nm. In some embodiments, about 95% of the aggregate volume of all nanoparticles in a nanoparticle composition comprises or consists of nanoparticles having diameters between 10 nm and 120 nm. In some embodiments, about 95% of the aggregate volume of all particles in a provided composition comprises or consists of nanoparticles having diameters between 10 nm and 150 nm.

In some embodiments, nanoparticle compositions are or comprise emulsions or dispersions. In some embodiments, nanoparticle compositions are "oil-in-water" dispersions (i.e., dispersions in which oily particles are dispersed within an aqueous dispersion medium); in some embodiments, nanoparticle compositions are "water-in-oil" dispersions (i.e., dispersions in which aqueous particles are dispersed within an oily dispersion medium).

In some embodiments, provided compositions do not require toxic solvents. By contrast, many conventional strategies for inducing formation of nanoparticles in a composition utilize toxic (typically organic) solvents. In some embodiments, provided compositions do not require polymers. By contrast, many conventional strategies for preparing compositions that contain nanoparticle structures require polymers.

In some embodiments, provided compositions have better tissue absorption and/or better biocompatibility than other nanoparticle compositions. For example, in some embodiments, provided compositions have better tissue absorption and/or better biocompatibility than nanoparticle compositions that are not uniform, that utilize one or more toxic (e.g., organic) solvents, and/or that utilize one or more polymers.

In some embodiments, nanoparticle compositions are stable. In some embodiments, a stable nanoparticle composition is one for which the average particle size, the maximum particle size, the range of particle sizes, and/or the distribution of particle sizes (i.e., the percentage of particles above a designated size and/or outside a designated range of sizes) is maintained for a period of time. In some embodiments, the period of time is at least about one hour; in some embodiments the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, or longer. In some embodiments, the period of time is within the range of about one (1) day to about twenty-four (24) months, about two (2) weeks to about twelve (12) months, about two (2) months to about five (5) months, etc. For example, if a population of nanoemulsion particles is subjected to prolonged storage, temperature changes, and/or pH changes and a majority of the nanoparticles in the population maintain a diameter within a stated range (i.e., for example, between approximately 10 nm and about 120 nm), the nanoparticle composition is stable. For some such populations, a majority is more than about 50%, about 60%, about 70%, about 80%, about 90%, about 95%, about 96%, about 97%, about 98%, about 99%, about 99.5%, about 99.6%, about 99.7%, about 99.8%, about 99.9%, or more than about 99.9% pure. In some embodiments, where a nanoparticle composition comprises at least one known therapeutic agent and/or an independently active biologically active agent, the nanoparticle composition is considered stable if the concentration of the known therapeutic agent and/or an independently active biologically active agent (e.g., PAI-1 inhibitors) is maintained in the composition over the designated period of time under a designated set of conditions.

As described herein, provided compositions are useful in various cosmetic and/or medical applications. Such compositions may be administered to a subject by any appropriate route, as may be readily determined by those skilled in the art for the disease, disorder, or condition of interest. In some embodiments, routes that may be employed may include one or more of oral (PO), intravenous (IV), intramuscular (IM), intra-arterial, intramedullary, intrathecal, subcutaneous (SQ), intraventricular, transdermal, interdermal, intradermal, rectal (PR), vaginal, intraperitoneal (IP), intragastric (IG), topical and/or transdermal (e.g., by lotions, creams, powders, ointments, liniments, gels, drops, etc.), mucosal, intranasal, buccal, enteral, vitreal, and/or sublingual administration; by intratracheal instillation, bronchial instillation, and/or inhalation; as an oral spray, nasal spray, and/or aerosol, and/or through a portal vein catheter; and/or combinations of any of the foregoing. In most embodiments, as described herein, administration will be topical, parenteral, or oral.

Formulations of provided compositions may be prepared by any appropriate method, as will be understood in the art. In general, such preparatory methods include a step of bringing an provided composition into association with one or more excipients, and then, if necessary and/or desirable, shaping and/or packaging into an appropriate form for administration, for example as or in a single or multi-dose unit.

In some embodiments, compositions may be prepared, packaged, and/or sold in bulk, as a single unit dose, and/or as a plurality of single unit doses. As used herein, a "unit dose" is a discrete amount of a pharmaceutical composition comprising a predetermined amount of the provided composition. The amount of a provided composition is generally equal to the dosage of the provided composition which would be administered to a subject and/or a convenient fraction of such a dosage such as, for example, one-half or one-third of such a dosage.

In some embodiments, appropriate excipients for use in compositions (e.g., pharmaceutically and/or cosmetically acceptable compositions) may, for example, include one or more excipients such as solvents, dispersion media, granulating media, diluents, or other liquid vehicles, dispersion or suspension aids, surface active agents and/or emulsifiers, isotonic agents, thickening or emulsifying agents, preservatives, solid binders, lubricants, disintegrating agents, binding agents, preservatives, buffering agents and the like, as suited to the particular dosage form desired. In some embodiments, excipients such as cocoa butter and/or suppository waxes, coloring agents, coating agents, sweetening, flavoring, and/or perfuming agents can be utilized. Remington's The Science and Practice of Pharmacy, 21st Edition, A. R. Gennaro (Lippincott, Williams & Wilkins, Baltimore, MD, 2005) discloses various excipients used in formulating pharmaceutical compositions and known techniques for the preparation thereof.

In some embodiments, an appropriate excipient (e.g., a pharmaceutically and/or cosmetically acceptable excipient) is at least 95%, at least 96%, at least 97%, at least 98%, at least 99%, or 100% pure. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or other International Pharmacopoeia.

In some embodiments, provided compositions are formulated as a cream, liniment, ointment, oil, foam, spray, lotion, liquid, powder, thickening lotion, or gel (e.g., formulated for transdermal delivery as described herein). Particular exemplary such formulations may be prepared, for example, as cosmetic formulation products such as skin softeners, nutritional lotion type emulsions, cleansing lotions, cleansing creams, skin milks, emollient lotions, massage creams, emollient creams, make-up bases, facial packs or facial gels, cleaner formulations such as shampoos, rinses, body cleansers, hair-tonics, or soaps, or dermatological compositions such as lotions, ointments, gels, creams, liniments, patches, deodorants, or sprays.

The present disclosure encompasses the recognition that emulsion technologies can provide stabilization benefits to agents of interest, including to PAI-1 inhibitors as described herein. Furthermore, emulsions, both macro and nano, may be used to prepare formulations for administration of PAI-1 inhibitors as treatment for dermatological conditions. In some particular embodiments, formulations may be topical formulations. In some particular embodiments, formulations may be injectable formulations. In some particular embodiments, formulations may be oral formulations.

In some embodiments, provided compositions comprise provided nanoemulsion compositions. In some embodiments, provided compositions are cream and/or lotion formulations. In some embodiments, provided cream and/or lotion formulations comprise nanoemulsion compositions. In some embodiments, compositions comprise provided nanoemulsion compositions but are not cream and/or lotion formulations. In some embodiments, suitable compositions are formulated into creams and/or lotions but do not comprise a nanoemulsion composition.

In some embodiments, provided compositions comprise a mixture of a provided nanoemulsion composition and one or more pharmaceutically acceptable excipients, e.g., for topical and/or transdermal (e.g., by lotions, creams, powders, ointments, liniments, gels, drops, etc.) administration.

### Emulsions

In some embodiments, provided herein are surprisingly effective technologies for administration and delivery of PAI-1 inhibitors. In some embodiments, the present disclosure teaches topical, oral, and/or injectable formulations and compositions of such PAI-1 inhibitors for various dermatological conditions, including hair graying. In some embodiments, the present disclosure teaches methods of treating and/or preventing one or more dermatological conditions through the administration of PAI-1 inhibitor formulations and/or compositions to a subject in need thereof. In some embodiments, the formulations and/or compositions comprise emulsions.

Moreover, the present disclosure appreciates that certain liquid nanoemulsion technologies have been demonstrated to provide remarkable transdermal delivery attributes, even for very large molecules, such as botulinum and/or antibody agents. See, e.g., U.S. Patent Publication No. 2012/0328701, U.S. Patent Publication No. 2012/0328702, 8,318,181, and U.S. Patent No. 8,658,391. These liquid nanoemulsions are far superior to solid nanoparticle drug delivery, particularly transdermal drug delivery wherein, as noted by Gomaa, the solid nanoparticles cannot penetrate the skin but merely accumulate in the hair follicles. These liquid nanoemulsions are also stable for at least 34 months, making them a commercially viable from this perspective as well.

### Macroemulsions

In some embodiments, the present invention utilizes macroemulsion compositions comprising PAI-1 inhibitors that are particularly effective and/or useful in for therapeutic purposes of dermatological conditions, e.g. hair graying. In some embodiments, particular macroemulsion compositions are particularly effective and/or useful for topical, oral, and/or injectable administration of PAI-1 inhibitors to a subject in need thereof. In some embodiments macroemulsion compositions may comprise of one or more PAI-1 inhibitors.

In some embodiments, a macroemulsion may be formulated into a composition suitable for topical administration on the skin. In some embodiments, a composition suitable for topical administration may be a lotion, cream, powder, ointment, liniment, gel, or drops.

In some embodiments, macroemulsion formulations comprise water, medium chain triglyceride, span 65, polysorbate 80, methylparaben, and propylparaben. In some embodiments, macroemulsion formulations comprise water, medium chain triglyceride, span 65, and polysorbate 80.

In some embodiments, provided compositions comprise a mixture of a provided macroemulsion composition and one or more pharmaceutically acceptable excipients. In some embodiments, cream and/or lotion formulations comprise a mixture of a provided macroemulsion composition and/or a saline solution.

In some embodiments, provided compositions comprise macroemulsion compositions comprising one or more PAI-1 inhibitors. In some embodiments, provided compositions are cream and/or lotion formulations. In some embodiments, provided cream and/or lotion formulations comprise macroemulsion compositions. In some embodiments, compositions comprise provided macroemulsion compositions but are not cream and/or lotion formulations. In some embodiments, suitable compositions are formulated into creams and/or lotions but do not comprise a macroemulsion composition.

In some embodiments, provided compositions comprise a mixture of a provided macroemulsion composition and one or more pharmaceutically acceptable excipients, e.g., for topical and/or transdermal (e.g., by lotions, creams, powders, ointments, liniments, gels, drops, etc.) administration.

In some embodiments, a macroemulsion may be formulated into a composition suitable for topical administration. In some embodiments, a composition suitable for topical administration may be a lotion, cream, powder, ointment, liniment, gel, or drops. In some embodiments, a macroemulsion may be formulated into an injectable composition. In some embodiments, the injectable composition may be sterile.

Macroemulsion formulations may act to stabilize the active agent and/or therapeutic agent such as PAI-1 inhibitors. Macroemulsion formulations would not necessarily be expected in and of themselves to achieve transdermal delivery of the active agents, nonetheless, the present disclosure encompasses that stabilization improvement that may be provided by incorporation into a macroemulsion composition might, when combined with microneedling technologies as described herein, achieve synergistic enhancement of transdermal delivery.

### Nanoemulsions

In some embodiments, the present invention utilizes nanoemulsion compositions comprising PAI-1 inhibitors that are particularly effective and/or useful in for therapeutic purposes of dermatological conditions, e.g. hair graying. In some embodiments, particular nanoemulsion compositions are particularly effective and/or useful for topical, oral, and/or injectable administration of PAI-1 inhibitors to a subject in need thereof. In some embodiments nanoemulsion compositions may comprise of one or more PAI-1 inhibitors.

In some embodiments, provided nanoemulsion compositions comprise oil and surfactant at a ratio ranging between about 0.1:1 to about 2:1. In some embodiments, provided nanoemulsion compositions comprise oil and surfactant at a ratio of about 0.1:1 to about 1:1. In some embodiments, provided nanoemulsion compositions comprise oil and surfactant at a ratio of about 0.5:1 to about 1:1. In some embodiments, provided nanoemulsion compositions comprise oil and surfactant at a ratio of about 0.5:1 to about 1:1.5. In some embodiments, provided nanoemulsion compositions comprise oil and surfactant at a ratio of about 0.1:1, about 0.15:1, about 0.2:1, about 0.25:1, about 0.3:1, about 0.35:1, about 0.4:1, about 0.45:1, about 0.5:1, about 0.5:1, about 0.55:1, about 0.6:1, about 0.65:1, about 0.7:1, about 0.75:1, about 0.8:1, about 0.85:1, about 0.9:1, about 0.95:1, or about 1:1 In some embodiments, provided nanoemulsion compositions comprise oil and surfactant at a ratio of about 0.67:1.

In some embodiments, the aqueous dispersion medium (e.g., water, buffer, salt solution, etc.) and surfactant are utilized at a ratio ranging between 0.01 and 20. In some embodiments, the aqueous dispersion medium (e.g., water, buffer, salt solution, etc.) and surfactant are utilized at a ratio ranging between 0.1 and 20. In some embodiments, the aqueous dispersion medium (e.g., water, buffer, salt solution, etc.) and surfactant are utilized at a ratio ranging between 0.5 and 10. In some embodiments, the aqueous dispersion medium (e.g., water, buffer, salt solution, etc.) and surfactant are utilized at a ratio ranging between 0.5 and 1. In some embodiments, the ratio of aqueous dispersion medium (e.g., water, buffer, salt solution, etc.) to surfactant is approximately 0.01:1, approximately 0.02:1, approximately 0.03:1, approximately 0.04:1, approximately 0.05:1, approximately 0.06:1, approximately 0.07:1, approximately 0.08:1, approximately 0.0:1, approximately 0.1:1, approximately 0.2:1, approximately 0.3:1, approximately 0.4:1, approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to water is approximately 0.5:1, approximately 1:1, approximately 2:1, approximately 3:1, approximately 4:1, approximately 5:1, approximately 6:1, approximately 7:1, approximately 8:1, approximately 9:1, approximately 10:1, approximately 11:1, approximately 12:1, approximately 13:1, approximately 14:1, approximately 15:1, approximately 16:1, approximately 17:1, approximately 18:1, approximately 19:1, or approximately 20:1. In some embodiments, aqueous dispersion medium (e.g., water, buffer, salt solution, etc.) and surfactant are utilized at a ratio ranging between 0.5 and 2. In some embodiments, the ratio of aqueous dispersion medium (e.g., water, buffer, salt solution, etc.) to surfactant is approximately 0.5:1, approximately 1:1, or approximately 2:1. In some embodiments, the ratio of surfactant to aqueous dispersion medium (e.g., water, buffer, salt solution, etc.) is approximately 0.5:1, approximately 1:1, or approximately 2:1. In some embodiments, the ratio of aqueous dispersion medium (e.g., water, buffer, salt solution, etc.) to surfactant is approximately 1:1. In some embodiments, compositions utilizing such ratios of aqueous dispersion medium (e.g., water, buffer, salt solution, etc.) to surfactant comprise water-in-oil emulsions.

In some embodiments, droplets within nanoemulsion compositions have diameters (e.g., average and/or median diameters) within a range of about 10 nm to about 300 nm, about 10 nm to about 200 nm, about 10 nm to about 150 nm, about 10 nm to about 130 nm, about 10 nm to about 120 nm, about 10 nm to about 115 nm, about 10 nm to about 110 nm, about 10 nm to about 100 nm, or about 10 nm to about 90 nm. In some embodiments, droplets within nanoemulsion compositions have diameters (e.g., average and/or median diameters) within a range of 1 nm to 300 nm, 1 nm to 200 nm, 1 nm to 150 nm, 1 nm to 120 nm, 1 nm to 100 nm, 1 nm to 75 nm, 1 nm to 50 nm, or 1 nm to 25 nm. In some embodiments, droplets within nanoemulsion compositions have diameters (e.g., average and/or median diameters) of 1 nm to 15 nm, 15 nm to 200 nm, 25 nm to 200 nm, 50 nm to 200 nm, or 75 nm to 200 nm.

In some embodiments, a total droplet distribution is encompassed within a specified range of droplet diameter size. In some embodiments, less than 50%, 25%, 10%, 5%, or 1% of a total droplet distribution is outside of a specified range of droplet diameter sizes. In some embodiments, less than 1% of a total droplet distribution is outside of a specified range of droplet diameter sizes. In some embodiments, a nanoemulsion composition is substantially free of droplets having a diameter larger than 300 nm, 250 nm, 200 nm, 150 nm, 120 nm, 100 nm, 75 nm, 50 nm, or 25 nm. In some embodiments, less than 50%, 25%, 10%, 5%, or 1% of a total droplet distribution have diameters larger than 300 nm, 250 nm, 200 nm, 150 nm, 120 nm, 100 nm, 75 nm, 50 nm, or 25 nm.

In some embodiments, droplets within nanoemulsion compositions have an average droplet size that is under about 300 nm, about 250 nm, about 200 nm, about 150 nm, about 130 nm, about 120 nm, about 115 nm, about 110 nm, about 100 nm, about 90 nm, or about 50 nm. In some embodiments, average droplet size is within a range of about 10 nm and about 300 nm, about 50 nm and about 250, about 60 nm and about 200 nm, about 65 nm and about 150 nm, or about 70 nm and about 130 nm. In some embodiments, average droplet size is about 80 nm and about 110 nm. In some embodiments, average droplet size is about 90 nm and about 100 nm.

[0159] In some embodiments, nanoemulsion droplets have a zeta potential ranging between -80 mV and +80 mV. In some embodiments, nanoemulsion droplets have a zeta potential ranging between -50 mV and +50 mV. In some embodiments, nanoemulsion droplets have a zeta potential ranging between -25 mV and +25 mV. In some embodiments, nanoemulsion droplets have a zeta potential ranging between n -10 mV and +10 mV. In some embodiments, nanoemulsion droplets have a zeta potential of about -80 mV, about -70 mV, about -60 mV, about 50 mV, about -40 mV, about -30 mV, about -25 mV, about -20 mV, about -15 mV, about -10 mV, or about -5 mV. In some embodiments, nanoemulsion droplets have a zeta potential of about +50 mV, about +40 mV, about +30 mV, about +25 mV, about +20 mV, about +15 mV, about +10 mV, or about +5 mV. In some embodiments, nanoemulsion droplets have a zeta potential that is about 0 mV

In some embodiments, aqueous dispersion media and surfactant are utilized at a ratio ranging between about 8:1 and about 9:1. In some embodiments, aqueous dispersion media and surfactant are utilized at a ratio of about 8:1, about 8.1:1, about 8.2:1, about 8.3:1, about 8.4:1, about 8.5:1, about 8.6:1, about 8.7:1, about 8.8:1, about 8.9:1, about 9:1, etc. In some embodiments, aqueous dispersion media and surfactant are utilized at a ratio of about 8.7:1. In some embodiments, aqueous dispersion media and surfactant are utilized at a ratio of about 8.8:1.

In some embodiments, aqueous dispersion media and oil are utilized at a ratio ranging between about 12:1 and about 14:1. In some embodiments, aqueous dispersion media and surfactant are utilized at a ratio of about 12:1, about 12.1:1, about 12.2:1, about 12.3:1, about 12.4:1, about 12.5:1, about 12.6:1, about 12.7:1, about 12.8:1, about 12.9:1, about 13:1, about 13.1:1, about 13.2:1, about 13.3:1, about 13.4:1, about 13.5:1, about 13.6:1, about 13.7:1, about 13.8:1, about 13.9:1, about 14:1, etc. In some embodiments, aqueous dispersion media and surfactant are utilized at a ratio of about 13.1:1.

In some embodiments, the percent of oil in the nanoemulsion ranges between 0% and 50%. In some embodiments, the percent of oil in the nanoemulsion ranges between 0% and 40%. In some embodiments, the percent of oil in the nanoemulsion ranges between 0% and 30%. In some embodiments, the percent of oil in the nanoemulsion ranges between 0% and 20%. In some embodiments, the percent of oil in the nanoemulsion ranges between 0% and 10%. In some embodiments, the percent of oil in the nanoemulsion ranges between 0% and 5%. In some embodiments, the percent of oil in the nanoemulsion ranges between 5% and 10%, between 10% and 15%, between 15% and 20%, between 20% and 25%, between 25% and 30%, between 35% and 40%, or between 45% and 50%. In some embodiments, the percent of oil in the nanoemulsion ranges between 10% and 20%, between 10% and 30%, between 10% and 40%, or between 10% and 50%. In some embodiments, the percent of oil in the nanoemulsion ranges between 20% and 30%, between 20% and 40%, between 20% and 50%. In some embodiments, the percent of oil in the nanoemulsion ranges between 30% and 40% or between 30% and 50%. In some embodiments, the percent of oil in the nanoemulsion ranges between 40% and 50%.

In some embodiments the percent of oil is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 31%, approximately 32%, approximately 33%, approximately 34%, approximately 35%, approximately 36%, approximately 37%, approximately 38%, approximately 39%, approximately 40%, approximately 41%, approximately 42%, approximately 43%, approximately 44%, approximately 45%, approximately 46%, approximately 47%, approximately 48%, approximately 49%, or approximately 50%. In some embodiments the percent of oil is approximately 10%. In some embodiments the percent of oil is approximately 9%. In some embodiments the percent of oil is approximately 8%. In some embodiments the percent of oil is approximately 7%. In some embodiments the percent of oil is approximately 6%. In some embodiments the percent of oil is approximately 5%. In some embodiments the percent of oil is approximately 4%. In some embodiments the percent of oil is approximately 3%. In some embodiments the percent of oil is approximately 2%. In some embodiments the percent of oil is approximately 1%.

In some embodiments, nanoemulsion formulations comprise water, medium chain triglyceride, polysorbate 80, methylparaben, and propylparaben. In some embodiments, nanoemulsion formulations comprise water, medium chain triglyceride, and polysorbate 80.

In some embodiments, a nanoemulsion may be formulated into a composition suitable for topical administration. In some embodiments, a composition suitable for topical administration may be a lotion, cream, powder, ointment, liniment, gel, or drops. In some embodiments, a nanoemulsion may be formulated into an injectable composition. In some embodiments, the injectable composition may be sterile.

These compositions are particularly useful in that they can be used for delivery of agents to a subject in need thereof via topical and/or transdermal (e.g., by lotions, creams, powders, ointments, liniments, gels, drops, etc.) administration. In some embodiments, provided cream and/or lotion formulations may be administered to a subject in need thereof via topical and/or transdermal (e.g., by lotions, creams, powders, ointments, liniments, gels, drops, etc.) administration. In some embodiments, provided nanoemulsion compositions may be formulated into cream and/or lotion formulations. In some embodiments, provided cream and/or lotion formulations comprising nanoemulsion compositions may be useful and/or effective for topical administration to a subject. In some embodiments, provided nanoemulsion compositions may be admixed with one or more cream components in a cream formulation (e.g., a provided cream formulation) and/or a saline solution for preparation of a pharmaceutical composition.

The present invention encompasses the recognition that emulsion compositions (e.g., macroemulsion compositions and nanoemulsion compositions) may be formulated into cream and/or lotion formulations for administration to a subject. The present invention encompasses the recognition that provided cream and/or lotion formulations can be particularly useful for formulating emulsions, such as those described herein, for administration to a subject.

### Topical Formulations

Compositions as described herein are particularly useful in that they can be used for delivery of PAI-1 inhibitors to a subject in need thereof via topical and/or transdermal (e.g., by lotions, creams, powders, ointments, liniments, gels, drops, etc.) administration. In some embodiments, provided cream and/or lotion formulations comprising PAI-1 inhibitors are administered to a subject in need thereof via topical (e.g., by lotions, creams, powders, ointments, liniments, gels, drops, etc.) administration. In some embodiments, the topical formulations comprise macroemulsions, as described herein. In some embodiments the topical formulations comprise nanoemulsions, as described herein.

In some embodiments, cream and/or lotion formulations comprise purified water, methylparaben, mineral oil, isopropyl myristate, white petrolatum, emulsifying wax, and propylparaben. In some embodiments, cream and/or lotion formulations comprise purified water, mineral oil, isopropyl myristate, white petrolatum, and emulsifying wax.

In some embodiments, the present invention utilizes particular cream and/or lotion formulations as described herein. In some embodiments, provided cream and/or lotion formulations comprise water. In some embodiments, provided cream and/or lotion formulations comprise methylparaben. In some embodiments, provided cream and/or lotion formulations comprise mineral oil. In some embodiments, provided cream and/or lotion formulations comprise isopropyl myristate. In some embodiments, provided cream and/or lotion formulations comprise white petrolatum. In some embodiments, provided cream and/or lotion formulations comprise emulsifying wax. In some embodiments, provided cream and/or lotion formulations comprise propylparaben. In some embodiments, provided cream and/or lotion formulations do not comprise any parabens. In some embodiments, provided cream and/or lotion formulations do not comprise methylparaben. In some embodiments, provided cream and/or lotion formulations do not comprise propylparaben.

In some embodiments, cream and/or lotion formulations may be useful for topical and/or transdermal administration. The present invention encompasses the recognition that, in some embodiments, provided cream and/or lotion formulations can be particularly useful for delivery of PAI-1 inhibitors, for example, to the hair follicle located in the site of administration. In some embodiments, sites treated include those which used to have hair or hair follicles but no longer have hair or hair follicles. In some embodiments, provided cream and/or lotion formulations are formulated for topical delivery to a subject in need thereof. In some embodiments, provided cream and/or lotion formulations are administered to a subject in need thereof via topical delivery.

In some embodiments, provided compositions are formulated with cosmetically acceptable components. For example, in some embodiments, provided compositions are formulated with water and also any cosmetically acceptable solvent, in particular, monoalcohols, such as alkanols having 1 to 8 carbon atoms (like ethanol, isopropanol, benzyl alcohol and phenylethyl alcohol), polyalcohols, such as alkylene glycols (like glycerine, ethylene glycol and propylene glycol), and glycol ethers, such as mono-, di-, and tri-ethylene glycol monoalkyl ethers, for example, ethylene glycol monomethyl ether and diethylene glycol monomethyl ether, used singly or in a mixture. Such components can be present, for example, in proportions of up to as much as 60%, 70%, 80%, or 90% by weight, relative to the weight of the total composition.

In some embodiments, provided compositions for topical administration include one or more cosmetically acceptable components that impart appearance attributes desirable or appropriate for a subject to which the composition is to be administered (e.g., a matte appearance, which may be particularly desirable or appropriate for administration to subjects having greasy skin).

In some embodiments, provided compositions are formulated with at least one cosmetically acceptable filler material, for example, in order to obtain a matte product, which may be especially desired for individuals with greasy skin.

In some embodiments, one or more PAI-1 inhibitors are formulated into compositions suitable for topical administration. Exemplary PAI-1 inhibitors include those described herein. In some embodiments, provided compositions may be formulated and delivered in combination with microneedle skin conditioning (MSC) so that systemic delivery is achieved; in some embodiments, provided compositions may be formulated and/or delivered so that local, but not systemic, delivery is achieved.

In some embodiments, compositions suitable for topical formulation comprise a penetration enhancing agent. In some embodiments, a penetration enhancing agent degrades, disrupts and/or damages skin structure(s) and/or skin. In some embodiments, a penetration enhancing agent does not degrade, disrupt and/or damage skin structure(s) and/or skin. In some embodiments, a penetration enhancing agent is an irritant. In some embodiments, a penetration enhancing agent is not an irritant.

In some embodiments, the provided compositions may be incorporated into a device such as, for example, a patch. A variety of transdermal patch structures are known in the art; those of ordinary skill will appreciate that provided compositions may readily be incorporated into any of a variety of such structures. In some embodiments, a transdermal patch may comprise a plurality of needles extending from one side of the patch that is administered to the skin.

Those of ordinary skill in the art will appreciate that provided compositions may be incorporated into a device such as, for example, a patch. A variety of transdermal patch structures are known in the art; those of ordinary skill will appreciate that provided compositions may readily be incorporated into any of a variety of such structures. In some embodiments, a transdermal patch may comprise a plurality of needles extending from one side of the patch that is administered to the skin, wherein needles extend from the patch to project through the stratum corneum of the skin. In some embodiments, needles do not rupture a blood vessel. In some embodiments, needles do not penetrate deeply enough to reach nerves in the dermis of the skin.

In some embodiments, a transdermal patch includes an adhesive. Some examples of adhesive patches are well known (for example, see U.S. Design Patent 296,006; and U.S. Patents 6,010,715; 5,591,767; 5,008,110; 5,683,712; 5,948,433; and 5,965,154). Adhesive patches are generally characterized as having an adhesive layer, which will be administered to a patient's skin, a depot or reservoir for holding a provided composition, and an exterior surface that prevents leakage of the provided composition from the depot. The exterior surface of a patch may be non-adhesive.

In accordance with the present invention, a provided composition is incorporated into a patch so that it remains stable for extended periods of time. For example, in some embodiments, a provided composition may be incorporated into a polymeric matrix that stabilizes an active agent, and permits the agent to diffuse from the matrix and the patch. A provided composition may also be incorporated into an adhesive layer of a patch so that once the patch is administered to the skin, the provided composition may diffuse through the skin. In some embodiments, an adhesive layer may be heat-activated where temperatures of about 37 °C cause the adhesive to slowly liquefy so that the agent diffuses through the skin. The adhesive may remain tacky when stored at less than 37°C, and once administered to the skin, the adhesive loses its tackiness as it liquefies.

In some embodiments, a provided composition can be provided in a depot in a patch so that pressure applied to the patch causes the provided composition to be directed out of the patch through microneedles and through the stratum corneum. Exemplary embodiments of microneedles are described above. Suitable devices for use in administering provided compositions intradermally include devices such as those described in U.S. Patent Nos. 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof.

In some embodiments, for example in order to prolong the effect of a provided composition, it may be desirable to slow absorption of a provided composition into the skin. In some embodiments, this may be accomplished by use of a liquid suspension of crystalline or amorphous material with poor water solubility. The rate of absorption of a provided composition then depends upon its rate of dissolution which, in turn, may depend upon crystal size and crystalline form. In some embodiments, depending upon the ratio of provided composition to polymer and the nature of the particular polymer employed, the rate of provided composition release can be controlled. Examples of other biodegradable polymers include poly(orthoesters) and poly(anhydrides).

### Injectable Formulations

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables.

Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of a provided composition, it may be desirable to slow the absorption of the provided composition from subcutaneous or intramuscular injection. In some embodiments, administration achieves systemic delivery. In some embodiments, administration achieves local delivery. This may be accomplished by the use of a liquid suspension of crystalline or amorphous material with poor water solubility. The rate of absorption of the provided composition then depends upon its rate of dissolution which, in turn, may depend upon crystal size and crystalline form. Alternatively, delayed absorption of a parenterally administered provided composition form is accomplished by dissolving or suspending the provided composition in an oil vehicle. Injectable depot forms are made by forming microencapsule matrices of the provided composition in biodegradable polymers such as polylactide-polyglycolide. Depending upon the ratio of provided composition to polymer and the nature of the particular polymer employed, the rate of provided composition release can be controlled. Examples of other biodegradable polymers include poly(orthoesters) and poly(anhydrides). Depot injectable formulations are prepared by entrapping the provided composition in liposomes or microemulsions which are compatible with body tissues.

### Oral Formulations

Solid dosage forms for oral administration include capsules, tablets, pills, powders, and granules. In such solid dosage forms, the provided composition is mixed with at least one inert, pharmaceutically acceptable excipient such as sodium citrate or dicalcium phosphate and/or fillers or extenders (*e.g*., starches, lactose, sucrose, glucose, mannitol, and silicic acid), binders (*e.g*., carboxymethylcellulose, alginates, gelatin, polyvinylpyrrolidinone, sucrose, and acacia), humectants (*e.g*., glycerol), disintegrating agents (*e.g.*, agar, calcium carbonate, potato starch, tapioca starch, alginic acid, certain silicates, and sodium carbonate), solution retarding agents (*e.g*., paraffin), absorption accelerators (*e.g.,* quaternary ammonium compounds), wetting agents (*e.g.,* cetyl alcohol and glycerol monostearate), absorbents (*e.g.,* kaolin and bentonite clay), and lubricants (*e.g.,* talc, calcium stearate, magnesium stearate, solid polyethylene glycols, sodium lauryl sulfate), and mixtures thereof. In the case of capsules, tablets and pills, the dosage form may comprise buffering agents.

Solid compositions of a similar type may be employed as fillers in soft and/or hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the provided composition(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polyethylene glycols and the like.

### Administration

The present disclosure provides technologies for treating dermatological conditions or disorders, including hair graying, using any of the provided compositions (*e.g.*, provided emulsion compositions; cream and/or lotion formulations; combination of provided emulsion compositions and cream and/or lotion formulation; *etc*.) as described herein. In some embodiments, the provided compositions are administered in combination with MSC.

As described herein, the present disclosure provides methods of administering provided compositions to a subject for various applications including, for example, cosmetic and/or medical applications. In some embodiments, the present disclosure provides methods of treating and/or preventing diseases, disorders, and/or conditions associated with activity of epidermal and/or dermal structures (*e.g*., sweat glands, sebaceous glands, hair follicles, *etc*.) by administering provided compositions to a subject in need thereof. However, only a non-therapeutic cosmetic process for treating or preventing hair graying is claimed.

### Site

According to the present disclosure, a PAI-1 inhibitor can be administered to a site of interest for treatment and/or prevention of a dermatologic condition at the site.

Technologies of the disclosure are suitable for both human and veterinary use. In some embodiments, subjects suffering from any dermatological disorder described herein, which would benefit from topical, oral, and/or injectable administration of a PAI-1 inhibitor may be treated with the disclosed technologies.

Any site suitable site for MSC is a suitable administration site. In some embodiments, an administration site is the skin overlying a muscle or muscle group of a subject. In some embodiments, the site is hairless. In some embodiments, the site is on the torso. In some embodiment the site is on the back. In some embodiments the site is on the chest. In some embodiments, the site is on the buttocks. In some embodiments, the site is on the crotch. In some embodiments, the site is on the groin. In some embodiments, the site is on the head. In some embodiments the site is on the scalp. In some embodiments, the site is on the face. In some embodiments the site is on the neck. In some embodiments the site is on the décolleté. In some embodiments, the site is in the armpit. In some embodiments, the site is on the axillae. In some embodiments the site is on the hands. In some embodiments the site is on the feet. In some embodiments the site is on the arms. In some embodiments the site is on the legs. In some embodiments, the site used to have hair or hair follicles but no longer have hair or hair follicles.

In some embodiments, the site of interest has hair follicles. In some embodiments, the hair follicles have normal structure and/or density. In some embodiments, the hair follicles do not comprise hairs. In some embodiments, the hair follicles comprise hairs. In some embodiments, percentage of hair follicles with hair is about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more.

In some embodiments, the hairs in the hair follicles are not gray in color. In some embodiments, the hairs in the hair follicles are gray in color. In some embodiments, percentage gray is about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more.

In some embodiments the site is affected by a dermatologic condition.

In some embodiments, the length of the microneedles used in MSC is adjusted based on skin thickness of the treatment site.

In some embodiments, MSC comprises one impression of microneedle (MN) or MN array. In some embodiments, MSC comprises two impressions of MN or MN array. In some embodiments, MSC comprises three impressions of MN or MN array. In some embodiments, MSC comprises four impressions of MN or MN array. In some embodiments, MSC comprises five impressions of MN or MN array. In some embodiments, MSC comprises six impressions of MN or MN array. In some embodiments, MSC comprises seven impressions of MN or MN array. In some embodiments, MSC comprises eight impressions of MN or MN array. In some embodiments, MSC comprises nine impressions of MN or MN array. In some embodiments, MSC comprises ten impressions of MN or MN array. In some embodiments, MSC comprises eleven impressions of MN or MN array. In some embodiments, MSC comprises twelve impressions of MN or MN array. In some embodiments, MSC comprises thirteen impressions of MN or MN array. In some embodiments, MSC comprises fourteen impressions of MN or MN array. In some embodiments, MSC comprises fifteen impressions of MN or MN array. In some embodiments, MSC comprises sixteen impressions of MN or MN array. In some embodiments, MSC comprises seventeen impressions of MN or MN array. In some embodiments, MSC comprises eighteen impressions of MN or MN array. In some embodiments, MSC comprises nineteen impressions of MN or MN array. In some embodiments, MSC comprises twenty impressions of MN or MN array. In some embodiments, the MSC comprises rolling the MN or MN array over the skin one or more times. In some embodiments, an MN array is rotated between impressions. In some embodiments an MN array is not rotated between impressions. In some embodiments impressions are made on the same site. In some embodiments impressions are made on overlapping sites. In some embodiments, impressions are made on different sites. In some embodiments, impressions are made by stamping of a MN array. In some embodiments, impressions are made by rolling a microneedle roller over a site one or more times. In accordance with established MN practices, in some embodiments, the MN array skin impressions last under one second or, alternatively, in some embodiments, they last over one second and may, for example, last for 30 seconds or more, 60 seconds or more, two minutes or more, five minutes or more, ten minutes or more, thirty minutes or more, etc.

### Subject

In general the subject is an organism, typically a mammal (e.g., a human, in some embodiments including prenatal human forms). In some embodiments, the subject is male. In some embodiments, subject is female. In some embodiments, the subject is human. In a particular embodiment the human subject is at least 10 years old. In some embodiments, the subject has no hair. In some embodiments, the subject has hair. In some embodiments the subject has low follicular density. In some embodiments, the subject has a high follicular density. In some embodiments, the subject has colored hair. In some embodiments, a subject is suffering from a relevant disease, disorder or condition (e.g. dermatological condition such as hair graying, etc.). In some embodiments, a subject is susceptible to a disease, disorder, or condition (e.g. dermatological condition such as hair graying, etc.). In some embodiments, a subject displays one or more symptoms or characteristics of a disease, disorder or condition (e.g. dermatological condition such as hair graying, etc.). In some embodiments, a subject does not display any symptom or characteristic of a disease, disorder, or condition (e.g. dermatological condition such as hair graying, etc.). In some embodiments, a subject is someone with one or more features characteristic of susceptibility to or risk of a disease, disorder, or condition (e.g. dermatological condition such as hair graying, etc.). In some embodiments, a subject is a patient. In some embodiments, a subject is an individual to whom diagnosis and/or therapy is and/or has been administered.

The technologies of the disclosure are suitable for both human and veterinary use. In some embodiments, subjects suffering from any dermatological disorder described herein, which would benefit from topical, oral, and/or injectable administration of a PAI-1 inhibitor may be treated with the disclosed technologies.

### Route

In general, route is selected to achieve delivery of a therapeutically effective amount to a relevant site of action. Without wishing to be bound by any particular theory, in some embodiments, a site of action may be or comprise a site comprising a hair follicle. In some embodiments, an administration site is the skin overlying a muscle or muscle group of a subject. In some embodiments, the site is hairless. In some embodiments, the site is on the torso. In some embodiment the site is on the back. In some embodiments the site is on the chest. In some embodiments, the site is on the buttocks. In some embodiments, the site is on the crotch. In some embodiments, the site is on the groin. In some embodiments, the site is on the head. In some embodiments the site is on the scalp. In some embodiments, the site is on the face. In some embodiments the site is on the neck. In some embodiments the site is on the décolleté. In some embodiments, the site is in the armpit. In some embodiments, the site is on the axillae. In some embodiments the site is on the hands. In some embodiments the site is on the feet. In some embodiments the site is on the arms. In some embodiments the site is on the legs.

In some embodiments, the present invention provides methods of administration of provided compositions via any route of delivery, including, but not limited to, oral (PO), intravenous (IV), intramuscular (IM), intra-arterial, intramedullary, intrathecal, subcutaneous (SQ), intraventricular, transdermal, interdermal, intradermal, rectal (PR), vaginal, intraperitoneal (IP), intragastric (IG), topical and/or transdermal (*e.g*., by lotions, creams, liniments, ointments, powders, gels, drops, *etc*.)*,* mucosal, intranasal, buccal, enteral, vitreal, and/or sublingual administration; by intratracheal instillation, bronchial instillation, and/or inhalation; as an oral spray, nasal spray, and/or aerosol, and/or through a portal vein catheter; and/or combinations thereof.

In some embodiments, provided methods involve topical, transdermal, or intradermal administration of provided compositions to the skin of a subject. In some embodiments, such routes achieve local delivery.

In some particular embodiments, provided method involves topical administration of an emulsion composition comprising PAI-1 inhibitors. In some particular embodiments, the emulsion composition is a macroemulsion. In some particular embodiments, the emulsion composition is a nanoemulsion. In some particular embodiments, topical via or in conjunction with MSC.

In some embodiments, an active agent or biologically active agent (e.g. PAI-1 inhibitor) penetrates the skin within about 1, 2, 3, 4, 5, 6, 7, 8, 9, or 10 minutes of administration. In some embodiments, a biologically active agent penetrates the skin within about 5 to about 60 minutes of administration. In some embodiments, a biologically active agent penetrates the skin within about 5 to about 12 minutes of administration. In some embodiments, a biologically active agent penetrates the skin within about 5 to about 15 minutes of administration. In some embodiments, a biologically active agent penetrates the skin within about 15 to about 30 minutes of administration. In some embodiments, a biologically active agent penetrates the skin within about 1 hour of administration. In some embodiments, a biologically active agent penetrates the skin within about 2 hours of administration. In some embodiments, a biologically active agent penetrates the skin within about 3 hours of administration. In some embodiments, a biologically active agent penetrates the skin within about 4 hours of administration. In some embodiments, a biologically active agent penetrates the skin within about 5 hours of administration. In some embodiments, a biologically active agent penetrates the skin within about 6 hours of administration. In some embodiments, a biologically active agent penetrates the skin within about 7 hours of administration. In some embodiments, a biologically active agent penetrates the skin within about 8 hours of administration. In some embodiments, a biologically active agent penetrates the skin within about 12 hours of administration. In some embodiments, a biologically active agent penetrates the skin within about 24 hours of administration.

In some embodiments, a biologically active agent (e.g., PAI-1 inhibitor) penetrates a layer of the skin within about 1, 2, 3, 4, 5, 6, 7, 8, 9, or 10 minutes of administration. In some embodiments, a biologically active agent penetrates a layer of the skin within about 5 to about 60 minutes of administration. In some embodiments, a biologically active agent penetrates a layer of the skin within about 5 to about 12 minutes of administration. In some embodiments, a biologically active agent penetrates a layer of the skin within about 5 to about 15 minutes of administration. In some embodiments, a biologically active agent penetrates a layer of the skin within about 15 to about 30 minutes of administration. In some embodiments, a biologically active agent penetrates a layer of the skin within about 1 hour of administration. In some embodiments, a biologically active agent penetrates a layer of the skin within about 2 hours of administration. In some embodiments, a biologically active agent penetrates a layer of the skin within about 3 hours of administration. In some embodiments, a biologically active agent penetrates a layer of the skin within about 4 hours of administration. In some embodiments, a biologically active agent penetrates a layer of the skin within about 5 hours of administration. In some embodiments, a biologically active agent penetrates a layer of the skin within about 6 hours of administration. In some embodiments, a biologically active agent penetrates a layer of the skin within about 7 hours of administration. In some embodiments, a biologically active agent penetrates a layer of the skin within about 8 hours of administration. In some embodiments, a biologically active agent penetrates a layer of the skin within about 12 hours of administration. In some embodiments, a biologically active agent penetrates a layer of the skin within about 24 hours of administration.

In some embodiments, a biologically active agent penetrates the top layer of the skin within about 1, 2, 3, 4, 5, 6, 7, 8, 9, or 10 minutes of administration. In some embodiments, a biologically active agent penetrates the top layer of the skin within about 5 to about 60 minutes of administration. In some embodiments, a biologically active agent penetrates the top layer of the skin within about 5 to about 12 minutes of administration. In some embodiments, a biologically active agent penetrates the top layer of the skin within about 5 to about 15 minutes of administration. In some embodiments, a biologically active agent penetrates the top layer of the skin within about 15 to about 30 minutes of administration. In some embodiments, a biologically active agent penetrates the top layer of the skin within about 1 hour of administration. In some embodiments, a biologically active agent penetrates the top layer of the skin within about 2 hours of administration. In some embodiments, a biologically active agent penetrates the top layer of the skin within about 3 hours of administration. In some embodiments, a biologically active agent penetrates the top layer of the skin within about 4 hours of administration. In some embodiments, a biologically active agent penetrates the top layer of the skin within about 5 hours of administration. In some embodiments, a biologically active agent penetrates the top layer of the skin within about 6 hours of administration. In some embodiments, a biologically active agent penetrates the top layer of the skin within about 7 hours of administration. In some embodiments, a biologically active agent penetrates the top layer of the skin within about 8 hours of administration. In some embodiments, a biologically active agent penetrates the top layer of the skin within about 12 hours of administration. In some embodiments, a biologically active agent penetrates the top layer of the skin within about 24 hours of administration.

In some embodiments, a biologically active agent penetrates the top layer of the skin, including the stratum corneum, dermal pores, hair follicles, and/or dermal glands within about 1, 2, 3, 4, 5, 6, 7, 8, 9, or 10 minutes of administration. In some embodiments, a biologically active agent penetrates the top layer of the skin, including the stratum corneum, dermal pores, hair follicles, and/or dermal glands within about 5 to about 60 minutes of administration. In some embodiments, a biologically active agent penetrates the top layer of the skin, including the stratum corneum, dermal pores, hair follicles, and/or dermal glands within about 5 to about 12 minutes of administration. In some embodiments, a biologically active agent penetrates the top layer of the skin, including the stratum corneum, dermal pores, hair follicles, and/or dermal glands within about 5 to about 15 minutes of administration. In some embodiments, a biologically active agent penetrates the top layer of the skin, including the stratum corneum, dermal pores, hair follicles, and/or dermal glands within about 15 to about 30 minutes of administration. In some embodiments, a biologically active agent penetrates the top layer of the skin, including the stratum corneum, dermal pores, hair follicles, and/or dermal glands within about 1 hour of administration. In some embodiments, a biologically active agent penetrates the top layer of the skin, including the stratum corneum, dermal pores, hair follicles, and/or dermal glands within about 2 hours of administration. In some embodiments, a biologically active agent penetrates the top layer of the skin, including the stratum corneum, dermal pores, hair follicles, and/or dermal glands within about 3 hours of administration. In some embodiments, a biologically active agent penetrates the top layer of the skin, including the stratum corneum, dermal pores, hair follicles, and/or dermal glands within about 4 hours of administration. In some embodiments, a biologically active agent penetrates the top layer of the skin, including the stratum corneum, dermal pores, hair follicles, and/or dermal glands within about 5 hours of administration. In some embodiments, a biologically active agent penetrates the top layer of the skin, including the stratum corneum, dermal pores, hair follicles, and/or dermal glands within about 6 hours of administration. In some embodiments, a biologically active agent penetrates the top layer of the skin, including the stratum corneum, dermal pores, hair follicles, and/or dermal glands within about 7 hours of administration. In some embodiments, a biologically active agent penetrates the top layer of the skin, including the stratum corneum, dermal pores, hair follicles, and/or dermal glands within about 8 hours of administration. In some embodiments, a biologically active agent penetrates the top layer of the skin, including the stratum corneum, dermal pores, hair follicles, and/or dermal glands within about 12 hours of administration. In some embodiments, a biologically active agent penetrates the top layer of the skin, including the stratum corneum, dermal pores, hair follicles, and/or dermal glands within about 24 hours of administration.

### Regimen

In general, regimen is selected to achieve delivery of a therapeutically effective amount to a relevant site of action. In some embodiments the compositions and formulations described herein may be administered to a subject in need thereof at a relevant site of action in a single dose. In some embodiments the compositions and formulations described herein may be administered to a subject in need thereof a relevant site of action in multiple doses. For example, the compositions and formulations described herein, can be administered through any one of the multiple routes of administration described herein (e.g. topical, oral, via injection) sufficient to achieve delivery of effective amount of the biologically active agent (e.g., PAI-1 inhibitor).

In some embodiments, a dosing regimen for a particular active agent (e.g., one or more PAI-1 inhibitors) may involve intermittent or continuous (*e.g*., by perfusion or other slow release system) administration, for example to achieve a particular desired pharmacokinetic profile or other pattern of exposure in one or more tissues or fluids of interest in the subject receiving therapy.

In some embodiments, different agents administered in combination may be administered via different routes of delivery and/or according to different schedules. Alternatively or additionally, in some embodiments, one or more doses of a first active agent is administered substantially simultaneously with, and in some embodiments via a common route and/or as part of a single composition with, one or more other active agents.

Factors to be considered when optimizing routes and/or dosing schedule for a given therapeutic regimen may include, for example, the particular indication being treated, the clinical condition of a subject (*e.g*., age, overall health, prior therapy received and/or response thereto) the site of delivery of the agent, the nature of the agent (*e.g.* an antibody or other polypeptide-based compound), the mode and/or route of administration of the agent, the presence or absence of combination therapy, and other factors known to medical practitioners. For example, in the treatment of cancer, relevant features of the indication being treated may include, for example, one or more of cancer type, stage, location.

In some embodiments, one or more features of a particular pharmaceutical composition and/or of a utilized dosing regimen may be modified over time (*e.g*., increasing or decreasing the amount of active agent in any individual dose, increasing or decreasing time intervals between doses), for example in order to optimize a desired therapeutic effect or response (*e.g*., inhibition of the PAI-1 gene or gene product).

In general, type, amount, and frequency of dosing of active agents in accordance with the present invention are governed by safety and efficacy requirements that apply when one or more relevant agent(s) is/are administered to a mammal, preferably a human. In general, such features of dosing are selected to provide a particular, and typically detectable, therapeutic response as compared to what is observed absent therapy.

In the context of the present disclosure, an exemplary desirable therapeutic response may involve, but is not limited to, inhibition of PAI-1 gene and/or gene product, inhibition and/or a reduction in the degree and/or prevalence of a relevant dermatologic condition. For example, dermatological conditions are those that are described herein. In a particular example, the dermatological condition is hair graying. Such criteria can be readily assessed by any of a variety of immunological, cytological, and other methods that are disclosed in the literature.

In some embodiments, an effective dose (and/or a unit dose) of an active agent, may be at least about 0.01 ng/kg body weight, at least about 0.01 µg/kg body weight, at least about 0.05 µg/kg body weight; at least about 0.1 µg/kg body weight, at least about 1 µg/kg body weight, at least about 2.5 µg/kg body weight, at least about 5 µg/kg body weight, at least about 10 µg /kg body weight, at least about 100 µg /kg body weight, at least about 1 mg /kg body weight, at least about 10 mg /kg body weight, at least about 100 mg /kg body weight, at least about 200 mg /kg body weight, at least about 300 mg /kg body weight, at least about 400 mg /kg body weight, and not more than about 500 mg/kg body weight. It will be understood by one of skill in the art that in some embodiments such guidelines may be adjusted for the molecular weight of the active agent. The dosage may also be varied for route of administration, the cycle of treatment, or consequently to dose escalation protocol that can be used to determine the maximum tolerated dose and dose limiting toxicity (if any) in connection to the administration of the PAI-1 antagonist and/or an additional therapeutic agent at increasing doses. Consequently, the relative amounts of the each agent within a pharmaceutical composition may also vary, for example, each composition may comprise between 0.001 % and 100% (w/w) of the corresponding agent.

In some embodiments, a "therapeutically effective amount" or "therapeutically effective dose" is an amount of a PAI-1 antagonist, or a combination of two or more PAI-1 antagonists, or a combination of a PAI-1 antagonist with one or more additional therapeutic agent(s), which inhibits, totally or partially, the progression of the condition or alleviates, at least partially, one or more symptoms of the condition. In some embodiments, a therapeutically effective amount can be an amount which is prophylactically effective. In some embodiments, an amount which is therapeutically effective may depend upon a patient's size and/or gender, the condition to be treated, severity of the condition and/or the result sought. In some embodiments, a therapeutically effective amount refers to that amount of a PAI-1 antagonist that results in amelioration of at least one symptom in a patient. In some embodiments, for a given patient, a therapeutically effective amount may be determined by methods known to those of skill in the art.

In some embodiments, toxicity and/or therapeutic efficacy of PAI-1 antagonists can be determined by standard pharmaceutical procedures in cell cultures or experimental animals, e.g., for determining the maximum tolerated dose (MTD) and the ED₅₀ (effective dose for 50% maximal response). Typically, the dose ratio between toxic and therapeutic effects is the therapeutic index; in some embodiments, this ratio can be expressed as the ratio between MTD and ED₅₀. Data obtained from such cell culture assays and animal studies can be used in formulating a range of dosage for use in humans.

In some embodiments, dosage may be guided by monitoring a PAI-1 antagonist's effect on one or more pharmacodynamic markers of inhibition in diseased or surrogate tissue. For example, cell culture or animal experiments can be used to determine the relationship between doses required for changes in pharmacodynamic markers and doses required for therapeutic efficacy can be determined in cell culture or animal experiments or early stage clinical trials. In some embodiments, dosage of a PAI-1 antagonist lies preferably within a range of circulating concentrations that include the ED₅₀ with little or no toxicity. In some embodiments, dosage may vary within such a range, for example depending upon the dosage form employed and/or the route of administration utilized. The exact formulation, route of administration and dosage can be chosen by the individual physician in view of the patient's condition. In the treatment of crises or severe conditions, administration of a dosage approaching the MTD may be required to obtain a rapid response.

In some embodiments, dosage amount and/or interval may be adjusted individually, for example to provide plasma levels of an active moiety which are sufficient to maintain, for example a desired effect, or a minimal effective concentration (MEC) for a period of time required to achieve therapeutic efficacy. In some embodiments, MEC for a particular PAI-1 antagonist can be estimated, for example, from *in vitro* data and/or animal experiments. Dosages necessary to achieve the MEC will depend on individual characteristics and route of administration. In some embodiments, high pressure liquid chromatography (HPLC) assays or bioassays can be used to determine plasma concentrations.

In some embodiments, dosage intervals can be determined using the MEC value. In certain embodiments, PAI-1 antagonists should be administered using a regimen which maintains plasma levels above the MEC for 10-90% of the time, preferably between 30-90% and most preferably between 50-90% until the desired amelioration of a symptom is achieved. In other embodiments, different MEC plasma levels will be maintained for differing amounts of time. In cases of local administration or selective uptake, the effective local concentration of the drug may not be related to plasma concentration.

One of skill in the art can select from a variety of administration regimens and will understand that an effective amount of a particular PAI-1 antagonist may be dependent on the subject being treated, on the subject's weight, the severity of the affliction, the manner of administration and/or the judgment of the prescribing physician.

In some embodiments, the present invention involves administration of at least one provided composition, administered according to a dosing regimen sufficient to achieve a reduction in the degree and/or prevalence of a relevant dermatologic condition of at least about 20%; in some embodiments according to a dosing regimen sufficient to achieve a of at least about 25%; in some embodiments according to a dosing regimen sufficient to achieve a reduction of at least about 30%; in some embodiments according to a dosing regimen sufficient to achieve a reduction of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more.

In some embodiments, the present invention involves administration of at least one provided composition, administered in combination with MSC, according to a dosing regimen sufficient to achieve a reduction in the degree and/or prevalence of a relevant dermatologic condition of at least about 20%; in some embodiments according to a dosing regimen sufficient to achieve a of at least about 25%; in some embodiments according to a dosing regimen sufficient to achieve a reduction of at least about 30%; in some embodiments according to a dosing regimen sufficient to achieve a reduction of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more.

In some embodiments, the present invention involves administration of at least one provided composition, administered optionally in combination with MSC, according to a dosing regimen sufficient to achieve a reduction in the degree and/or prevalence of a relevant dermatologic condition of at least about 20% in a specified percentage of a population of patients to which the composition was administered; in some embodiments according to a dosing regimen sufficient to achieve a of at least about 25% in a specified percentage of a population of patients to which the composition was administered; in some embodiments according to a dosing regimen sufficient to achieve a reduction of at least about 30% in a specified percentage of a population of patients to which the composition was administered; in some embodiments according to a dosing regimen sufficient to achieve a reduction of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more in a specified percentage of a population of patients to which the composition was administered. In some embodiments, the specified percentage of population of patients to which the composition was administered is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. To give but a few illustrative examples, in some embodiments, the present invention involves administration of at least one provided composition according to a dosing regimen sufficient to achieve a reduction in the degree and/or prevalence of a relevant dermatologic condition of at least about 20% in at least about 50% of the population of patients to which the composition was administered. In some embodiments, the present invention involves administration of at least one provided composition according to a dosing regimen sufficient to achieve a reduction in the degree and/or prevalence of a relevant dermatologic condition of at least about 30% in at least about 50% of the population of patients to which the composition was administered.

The present disclosure provides technologies for treating conditions or disorders by administering to a patient a provided composition as described herein (e.g., a provided emulsion composition; cream and/or lotion formulation; combination of provided emulsion composition and cream and/or lotion formulation; etc.), optionally in combination with MSC. In some embodiments, the present disclosure provides technologies for treating conditions or disorders by topically administering to a patient a composition containing a provided emulsion composition, optionally in combination with MSC as described herein.

In some embodiments, an active agent or biologically active agent (e.g. PAI-1 inhibitor) penetrates the skin within about 1, 2, 3, 4, 5, 6, 7, 8, 9, or 10 minutes of administration. In some embodiments, a biologically active agent penetrates the skin within about 5 to about 60 minutes of administration. In some embodiments, a biologically active agent penetrates the skin within about 5 to about 12 minutes of administration. In some embodiments, a biologically active agent penetrates the skin within about 5 to about 15 minutes of administration. In some embodiments, a biologically active agent penetrates the skin within about 15 to about 30 minutes of administration. In some embodiments, a biologically active agent penetrates the skin within about 1 hour of administration. In some embodiments, a biologically active agent penetrates the skin within about 2 hours of administration. In some embodiments, a biologically active agent penetrates the skin within about 3 hours of administration. In some embodiments, a biologically active agent penetrates the skin within about 4 hours of administration. In some embodiments, a biologically active agent penetrates the skin within about 5 hours of administration. In some embodiments, a biologically active agent penetrates the skin within about 6 hours of administration. In some embodiments, a biologically active agent penetrates the skin within about 7 hours of administration. In some embodiments, a biologically active agent penetrates the skin within about 8 hours of administration. In some embodiments, a biologically active agent penetrates the skin within about 12 hours of administration. In some embodiments, a biologically active agent penetrates the skin within about 24 hours of administration.

In some embodiments, a biologically active agent (e.g., PAI-1 inhibitor) penetrates a layer of the skin within about 1, 2, 3, 4, 5, 6, 7, 8, 9, or 10 minutes of administration. In some embodiments, a biologically active agent penetrates a layer of the skin within about 5 to about 60 minutes of administration. In some embodiments, a biologically active agent penetrates a layer of the skin within about 5 to about 12 minutes of administration. In some embodiments, a biologically active agent penetrates a layer of the skin within about 5 to about 15 minutes of administration. In some embodiments, a biologically active agent penetrates a layer of the skin within about 15 to about 30 minutes of administration. In some embodiments, a biologically active agent penetrates a layer of the skin within about 1 hour of administration. In some embodiments, a biologically active agent penetrates a layer of the skin within about 2 hours of administration. In some embodiments, a biologically active agent penetrates a layer of the skin within about 3 hours of administration. In some embodiments, a biologically active agent penetrates a layer of the skin within about 4 hours of administration. In some embodiments, a biologically active agent penetrates a layer of the skin within about 5 hours of administration. In some embodiments, a biologically active agent penetrates a layer of the skin within about 6 hours of administration. In some embodiments, a biologically active agent penetrates a layer of the skin within about 7 hours of administration. In some embodiments, a biologically active agent penetrates a layer of the skin within about 8 hours of administration. In some embodiments, a biologically active agent penetrates a layer of the skin within about 12 hours of administration. In some embodiments, a biologically active agent penetrates a layer of the skin within about 24 hours of administration.

In some embodiments, a biologically active agent penetrates the top layer of the skin within about 1, 2, 3, 4, 5, 6, 7, 8, 9, or 10 minutes of administration. In some embodiments, a biologically active agent penetrates the top layer of the skin within about 5 to about 60 minutes of administration. In some embodiments, a biologically active agent penetrates the top layer of the skin within about 5 to about 12 minutes of administration. In some embodiments, a biologically active agent penetrates the top layer of the skin within about 5 to about 15 minutes of administration. In some embodiments, a biologically active agent penetrates the top layer of the skin within about 15 to about 30 minutes of administration. In some embodiments, a biologically active agent penetrates the top layer of the skin within about 1 hour of administration. In some embodiments, a biologically active agent penetrates the top layer of the skin within about 2 hours of administration. In some embodiments, a biologically active agent penetrates the top layer of the skin within about 3 hours of administration. In some embodiments, a biologically active agent penetrates the top layer of the skin within about 4 hours of administration. In some embodiments, a biologically active agent penetrates the top layer of the skin within about 5 hours of administration. In some embodiments, a biologically active agent penetrates the top layer of the skin within about 6 hours of administration. In some embodiments, a biologically active agent penetrates the top layer of the skin within about 7 hours of administration. In some embodiments, a biologically active agent penetrates the top layer of the skin within about 8 hours of administration. In some embodiments, a biologically active agent penetrates the top layer of the skin within about 12 hours of administration. In some embodiments, a biologically active agent penetrates the top layer of the skin within about 24 hours of administration.

In some embodiments, a biologically active agent penetrates the top layer of the skin, including the stratum corneum, dermal pores, hair follicles, and/or dermal glands within about 1, 2, 3, 4, 5, 6, 7, 8, 9, or 10 minutes of administration. In some embodiments, a biologically active agent penetrates the top layer of the skin, including the stratum corneum, dermal pores, hair follicles, and/or dermal glands within about 5 to about 60 minutes of administration. In some embodiments, a biologically active agent penetrates the top layer of the skin, including the stratum corneum, dermal pores, hair follicles, and/or dermal glands within about 5 to about 12 minutes of administration. In some embodiments, a biologically active agent penetrates the top layer of the skin, including the stratum corneum, dermal pores, hair follicles, and/or dermal glands within about 5 to about 15 minutes of administration. In some embodiments, a biologically active agent penetrates the top layer of the skin, including the stratum corneum, dermal pores, hair follicles, and/or dermal glands within about 15 to about 30 minutes of administration. In some embodiments, a biologically active agent penetrates the top layer of the skin, including the stratum corneum, dermal pores, hair follicles, and/or dermal glands within about 1 hour of administration. In some embodiments, a biologically active agent penetrates the top layer of the skin, including the stratum corneum, dermal pores, hair follicles, and/or dermal glands within about 2 hours of administration. In some embodiments, a biologically active agent penetrates the top layer of the skin, including the stratum corneum, dermal pores, hair follicles, and/or dermal glands within about 3 hours of administration. In some embodiments, a biologically active agent penetrates the top layer of the skin, including the stratum corneum, dermal pores, hair follicles, and/or dermal glands within about 4 hours of administration. In some embodiments, a biologically active agent penetrates the top layer of the skin, including the stratum corneum, dermal pores, hair follicles, and/or dermal glands within about 5 hours of administration. In some embodiments, a biologically active agent penetrates the top layer of the skin, including the stratum corneum, dermal pores, hair follicles, and/or dermal glands within about 6 hours of administration. In some embodiments, a biologically active agent penetrates the top layer of the skin, including the stratum corneum, dermal pores, hair follicles, and/or dermal glands within about 7 hours of administration. In some embodiments, a biologically active agent penetrates the top layer of the skin, including the stratum corneum, dermal pores, hair follicles, and/or dermal glands within about 8 hours of administration. In some embodiments, a biologically active agent penetrates the top layer of the skin, including the stratum corneum, dermal pores, hair follicles, and/or dermal glands within about 12 hours of administration. In some embodiments, a biologically active agent penetrates the top layer of the skin, including the stratum corneum, dermal pores, hair follicles, and/or dermal glands within about 24 hours of administration.

### Penetration Enhancing Treatment

According to the present invention, in some embodiments, provided compositions may be administered in combination with one or more penetrating enhancing treatments (e.g. chemical agents, laser treatment, microneedling, physical massage etc.), such as known penetration enhancing agents and/or penetration enhancing treatment modalities, to for example, facilitating penetration of PAI-1 inhibitors across biological barrier (e.g., skin). In some embodiments, provided compositions include one or more such other penetration enhancing agents; in some embodiments, such other penetration enhancing agents are provided as part of distinct compositions. In some embodiments, penetration enhancing treatment involves simultaneous administration of two or more different penetration enhancing agents and/or penetration enhancing treatment modalities; in some embodiments, penetration enhancing treatment involves simultaneous exposure to two or more different penetration enhancing treatment agents and/or penetration enhancing treatment modalities, for example through simultaneous laser treatment and composition administration.

In some embodiments, penetration enhancing agents is or comprises chemical agents. For example, chemical agents that that may damage, disrupt, and/or degrade one or more stratum corneum components) may include, for example, alcohols, such as short chain alcohols, long chain alcohols, or polyalcohols; amines and amides, such as urea, amino acids or their esters, amides, AZONE^{®}, derivatives of AZONE^{®}, pyrrolidones, or derivatives of pyrrolidones; terpenes and derivatives of terpenes; fatty acids and their esters; macrocyclic compounds; tensides; or sulfoxides (e.g., dimethylsulfoxide (DMSO), decylmethylsulfoxide, etc.); surfactants, such as anionic, cationic, and nonionic surfactants; polyols; essential oils; and/or hyaluronidase. In some embodiments, a penetration enhancing agent may be an irritant in that an inflammatory and/or allergic reaction occurs when the agent is administered to skin. In some embodiments, a penetration enhancing agent is not an irritant. In some embodiments, a penetration enhancing agent may be or comprise a chemical agent that does not damage, disrupt, or degrade skin structure but whose presence or level nonetheless correlates with increased penetration of an agent of interest across skin, as compared with that observed in its absence. In some embodiments, co-peptides, carrier molecules, and carrier peptides may be penetration enhancing agents which do not damage, disrupt, and/or degrade skin structure(s). In some embodiments, co-peptides, carrier molecules, and carrier peptides may be penetration enhancing agents which do not irritate the skin. The term "penetration enhancing agent" does not encompass mechanical devices (e.g., needles, scalpels, etc.), or equivalents thereof (e.g., other damaging treatments). Also, those skilled in the art will appreciate that a structure such as a nanoparticle or an emulsion is not a chemical agent and therefore not a chemical penetration enhancing agent even if its presence correlates with enhanced skin penetration of an agent of interest that may be associated with the structure. In some embodiments, penetration enhancing agents is or comprises alcohol.

In some embodiments, penetration enhancing treatment modalities is or comprises microneedling. In some embodiments, penetration enhancing treatment modalities is or comprises laser treatment. In some embodiments, penetration enhancing treatment modalities is or comprises physical massage. For example, in some embodiments, the composition may be administered before or after a performing laser treatment of the site. In some embodiments, penetration enhancing treatment modalities is or comprises administration of an electric or magnetic field.

### Microneedling:

In some particular embodiments, microneedle (MN) arrays for use in accordance with the present disclosure are or share features with minimally invasive systems, developed to overcome some of the disadvantages commonly associated with the use of hypodermic and subcutaneous needles, as well as improve patient comfort and compliance. Such disadvantages include, for example, potential for needle tip misplacement with a hypodermic needle because a health professional cannot visualize where exactly the needle is going; such needle misplacement can result in adverse reactions when injected incorrectly. MN would be less prone to such a problem. Other advantages of MN are that they may not cause bleeding, minimize introduction of pathogens through MN produced holes, and eliminate transdermal dosing variability. Other advantages are the possibility of self-administration, reduce risk of accidental needle stick injuries, reduce risk of transmitting infection, and ease of disposal. In some embodiments, MN are multiple microscopic projections assembled on one side of a support, such as a patch or a device (e.g., stamp, roller, array, applicator, pen).

In some embodiments, MN for use in accordance with the present disclosure may be designed and/or constructed in arrays in order to improve skin contact and facilitate penetration into the skin. In some embodiments, utilized MN are of suitable length, width, and shape to minimize contact with nerves when inserted into the skin, while still creating efficient pathways for drug delivery. Alkilani, A. Z., et al., "Transdermal drug delivery: Innovative pharmaceutical developments based on disruption of the barrier properties of the stratum corneum." Pharmaceutics. 7:438-470 (2015).

In some embodiments, a suitable MN may be solid, coated, porous, dissolvable, hollow, or hydrogel MN. Solid MN create microholes in the skin, thereby increasing transport of a drug formulation (e.g., "poke and patch" methods). Coated MN allow for rapid dissolution of a coated drug into the skin (e.g., "coat and poke" methods). Dissolvable MN allow for rapid and/or controlled release of a drug incorporated within the microneedles. Hollow MN may be used to puncture the skin and enable release of a composition following active infusion or diffusion of a formulation through a microneedle's bores (e.g., "poke and flow" methods"). In the case of dissolvable MN, MN can act as a drug depot, holding a drug composition until released by dissolution in the case of dissolvable MN or swelling in the case of hydrogel MN (e.g., "poke and release" methods). However, as already described herein, in many embodiments, the active agent is not delivered by injection via one or more microneedles. That is, in many embodiments, any microneedle utilized in accordance with such embodiments is not coated, loaded, or fabricated with the biologically active agent in any way that would achieve delivery of the biologically active agent. Alternatively, in some embodiments, as described herein, a MN, utilized in accordance with the present disclosure (whether in MSC or otherwise), may comprise and/or deliver a biologically active agent, if the biologically active agent is formulated in a macro- or nano- emulsion composition as described herein. Thus, as will be appreciated by those skilled in the art reading the specification described herein, treatment of skin with microneedle(s) that deliver the biologically active agent (e.g., by injection through a microneedle, by the release of a microneedle coating or by the release from a dissolving microneedle) is not microneedle skin conditioning.

In some embodiments, a microneedle has a diameter which is consistent throughout the microneedle's length. In some embodiments, the diameter of a microneedle is greatest at the microneedle's base end. In some embodiments, a microneedle tapers to a point at the end distal to the microneedle's base. In some embodiments, a microneedle may be solid. In some embodiments, a microneedle may be hollow. In some embodiments a microneedle may be tubular. In some embodiments, a microneedle may be sealed on one end. In some embodiments, a microneedle is part of an array of microneedles. In some embodiments, a microneedle may have a length of between about 1 µm to about 4,000 µm. In some embodiments, a microneedle may have a length of between about 1 µm to about 2,000 µm. In some embodiments, a microneedle may have a length of between about 50 µm to about 400 µm. In some embodiments, a microneedle may have a length of between about 800 µm to about 1500 µm.

In some embodiments, MN for use in accordance with the present disclosure may be fabricated from different materials, using technologies including, but not limited to micro-molding processes or lasers. In some embodiments, MN may be manufactured using various types of biocompatible materials including polymers, metal, ceramics, semiconductors, organics, composites, or silicon. Unless they are designed to break off into the skin and dissolve, in some embodiments, microneedles have the mechanical strength to remain intact and to deliver drugs, or collect biological fluid, while being inserted into the skin and/or removed from the skin after insertion. In some embodiments MN are capable of remaining in place for up to a number of days before intact removal. In some embodiments, microneedles may be sterilizable using standard technologies. In some embodiments, MN are biodegradable. In some embodiments, MN comprise a polymeric material. In some embodiments the polymeric material comprises poly-L-lactic acid, poly-glycolic acid, polycarbonate, poly-lactic-co-glycolic acid (PLGA), polydimethylsiloxane, polyvinylpyrrolidone (PVP), a copolymer of methyl vinyl ether and maleic anhydride, sodium hyaluronate, carboxymethyl cellulose, maltose, dextrin, galactose, starch, gelatin, or a combination thereof.

Suitable MN arrays and MSC devices for use in combination with compositions comprising biologically active agents for transdermal delivery of biologically active agents include devices such as those described in e.g., U.S. Patents 6,334,856; 6,503,231; 6,908,453; 8,257,324; and 9,144,671.

### Combination Therapy or Treatment

According to the present disclosure, provided compositions may be administered in combination with one or more additional treatments. In some embodiments the one or more additional treatments is or comprises other active agents and/or therapeutic modalities (e.g. one or more PAI-inhibitors, or other agents), such as known therapeutic agents and/or independently active biologically active agents. In some embodiments, for example, provided compositions include one or more such other active agents; in some embodiments, such other active agents are provided as part of distinct compositions. In some embodiments, combination therapy involves simultaneous administration of one or more doses or units of two or more other active agents and/or therapeutic modalities; in some embodiments, combination therapy involves simultaneous exposure to two or more other active agents and/or therapeutic modalities, for example through overlapping dosing regimens. However, only a non-therapeutic cosmetic process for treating or preventing hair graying is claimed.

In some embodiments, provided compositions include or are administered in combination with one or more other active agents useful for the treatment of the relevant dermatologic or other disease, disorder and/or condition, for example as discussed herein in context of the relevant disease, disorder, and/or condition.

### Kits

In some embodiments, the present disclosure provides pharmaceutical packs or kits including one or more emulsion compositions comprising one or more PAI-1 inhibitors and/or one or more microneedle devices according to the present disclosure. In some embodiments, pharmaceutical packs or kits include preparations or pharmaceutical compositions containing provided compositions in one or more containers filled with optionally one or more additional ingredients of pharmaceutical compositions. In some embodiments, a pharmaceutical pack or kit includes an additional approved therapeutic agent for use in combination therapies. In some embodiments, optionally associated with such container(s) can be a notice in the form prescribed by a governmental agency regulating the manufacture, use or sale of pharmaceutical products, which notice reflects approval by the agency of manufacture, use, or sale for human administration.

Kits are provided that include therapeutic reagents and/or active agents, such as PAI-1 inhibitors. As but one non-limiting example, provided compositions can be provided as topical formulations and administered as therapy. Pharmaceutical doses or instructions therefor may be provided in a kit for administration to an individual suffering from or at risk for conditions or disorders, e.g., those associated with the dermal level of the skin.

In some embodiments, a kit may comprise (i) a provided composition; and (ii) at least one pharmaceutically acceptable excipient; and optionally (iii) at least one syringe, spatula, swab for administration to skin; and (iv) instructions for use.

In some embodiments, a kit may comprise (i) a provided composition; and (ii) at least one pharmaceutically acceptable excipient; and optionally (iii) a device for injection (e.g., syringe and needle, microneedle array, hair brush, etc.); and (iv) instructions for use.

It will be appreciated by those of ordinary skill in the art that inventive compositions for topical administration may have a cosmetic formulation such as skin softener, nutrition lotion type emulsion, cleansing lotion, cleansing cream, skin milk, emollient lotion, massage cream, emollient cream, make-up base, facial pack or facial gel, cleaner formulation such as shampoos, rinses, body cleanser, hair-tonics, or soaps, or dermatological composition such as lotions, ointments, gels, creams, patches or sprays. In some embodiments, compositions for topical administration are not formulated for administration to mucous membranes (e.g., are inappropriate for administration to mucous membranes and/or are not formulated to deliver an appropriate amount of large agent to or across mucous membranes).

### Exemplification

### Example 1: Effects of Topical PAI-1 inhibitor formulation on Hair Graying

A topical study of topical PAI-1 inhibitor formulation after topical administration of a topical formulation of PAI-1 inhibitor (e.g., see Table 1) in man is performed. The study is designed to test whether the topical formulation of PAI-1 inhibitor significantly reduces hair graying in man by measuring hair color change following topical treatment with a PAI-1 inhibitor.

The study includes two groups of 25 human subjects each. Both groups have subjects with varying levels of hair follicle density and varying numbers and/or density of gray hairs. The scalp of each subject in the second group is treated twice a day for 6 months topically with a fixed volume of a PAI-1 inhibitor formulation that is at a fixed concentration of the PAI-1 inhibitor. The concentration of the PAI-1 inhibitor in the formulation is 5% w/w. The administration of the topical preparation to the scalp takes about 5 minutes, after which the suspension is left on the site for about 8 to 12 hours. The scalps of the subjects in the first group are treated twice a day for 6 months topically with an empty formulation and is the control.

The expected effect of such a treatment is a reduction in the density of gray hairs at the site of the PAI-1 inhibitor formulation treatment. The number of gray hairs at the treatment sites is measured by two methods: 1) A photograph of the treated area is taken to observe a change in the hair color; or 2) A gray hair density test, wherein the gray hair count in a small site on each subject's scalp is measured. The small site on the scalp is selected prior to the commencement of the study.

A photograph and the gray hair density test method are employed at baseline prior to a PAI-1 inhibitor treatment. Following this photographs of the scalp of each subject is obtained every four weeks after start of treatment and at the end of the 6-month study; the gray hair density test is also performed every four weeks after start of treatment and at the end of the 6-month study. The study finds that at Baseline, the average amount of gray hairs counted by either the gray hair density tests or as observed from the photographs is approximately equal across the control and treatment groups. Photographs of the scalps of the subjects of the treatment group on average show a visual reduction in the total number of gray hairs with every four weeks. The gray hair density test also on average shows a reduction in the number of gray hairs in the site selected on the scalp of each subject belonging to the treatment group with every four weeks. In contrast subjects in the control group showed no visual reduction in gray hairs in the treatment site, or reduction in the density of gray hairs in the selected site on the scalp.

This study establishes that topical administration of the topical formulation of PAI-1 inhibitor reduces gray hair count and reverses hair graying in humans.

### Example 2: Effects of Oral PAI-1 inhibitor formulation on Hair Graying

A study of oral administration of an oral formulation of PAI-1 inhibitor in man is performed. The study is designed to test whether the oral formulation of PAI-1 inhibitor significantly reduces hair graying in man by measuring hair color change following oral treatment with a PAI-1 inhibitor.

The study includes two groups of 25 human subjects each. Both groups have subjects with varying levels of hair follicle density and varying numbers and/or density of gray hairs. Each subject in the second group is administered an oral preparation of PAI-1 inhibitor formulated as a 75 mg capsule thrice a day for 6 months. Each subject in the first group are administered an oral empty preparation formulated as a 75 mg capsule thrice a day for 6 months and serve as the control.

The expected effect of such a treatment is a reduction in the density of gray hairs due to the PAI-1 inhibitor formulation treatment. The number of gray hairs on the scalps of the subjects is measured by two methods: 1) A photograph of the scalp is taken to observe a change in the hair color; or 2) A gray hair density test, wherein the gray hair count in a small site on each subject's scalp is measured. The small site on the scalp is is selected prior to the commencement of the study.

A photograph and the gray hair density test method are employed at baseline prior to a PAI-1 inhibitor treatment. Following this photographs of the scalp of each subject is obtained every four weeks after start of treatment and at the end of the 6-month study; the gray hair density test is also performed every four weeks after start of treatment and at the end of the 6-month study. The study finds that at baseline, the average amount of gray hairs counted by either the gray hair density tests or as observed from the photographs is approximately equal across the control and treatment groups. Photographs of the scalps of the subjects of the treatment group on average show a visual reduction in the total number of gray hairs with every four weeks. The gray hair density test also on average shows a reduction in the number of gray hairs in the site selected on the scalp of each subject belonging to the treatment group with every four weeks. In contrast subjects in the control group showed no visual reduction in gray hairs in the treatment site, or reduction in the density of gray hairs in the selected site on the scalp.

This study establishes that oral administration of the oral formulation of PAI-1 inhibitor reduces gray hair count and reverses hair graying in humans.

### Example 3: Effects of Injectable PAI-1 inhibitor formulation on Hair Graying

A study of injectable PAI-1 inhibitor formulation after administration via injection of a sterile injectable formulation of PAI-1 inhibitor (e.g., see Table 1) in man is performed. The study is designed to test whether the injectable formulation of PAI-1 inhibitor significantly reduceshair graying in man by measuring hair color change following treatment with a PAI-1 inhibitor.

The study includes two groups of 25 human subjects each. Both groups have subjects with varying levels of hair follicle density and varying numbers and/or density of gray hairs. The scalp of each subject in the second group is treated twice a day for 6 months with a fixed volume of a PAI-1 inhibitor injectable formulation that is at a fixed concentration of the PAI-1 inhibitor. The concentration of the PAI-1 inhibitor in the formulation is 5% w/w. The administration of the injectable preparation to the scalp takes about 5-10 minutes, after which the suspension is left on the site for about 8 to 12 hours. The scalps of the subjects in the first group are treated twice a day for 6 months with an empty injectable formulation and is the control.

The expected effect of such a treatment is a reduction in the density of gray hairs due to the PAI-1 inhibitor formulation treatment. The number of gray hairs at the treatment sites is measured by two methods: 1) A photograph of the treated area is taken to observe a change in the hair color; or 2) A gray hair density test, wherein the gray hair count in a small site on each subject's scalp is measured. The small site on the scalp is selected prior to the commencement of the study.

A photograph and the gray hair density test method are employed at baseline prior to a PAI-1 inhibitor treatment. Following this photographs of the scalp of each subject is obtained every four weeks after start of treatment and at the end of the 6-month study; the gray hair density test is also performed every four weeks after start of treatment and at the end of the 6-month study. The study finds that at Baseline, the average amount of gray hairs counted by either the gray hair density tests or as observed from the photographs is approximately equal across the control and treatment groups. Photographs of the scalps of the subjects of the treatment group on average show a visual reduction in the total number of gray hairs with every four weeks. The gray hair density test also on average shows a reduction in the number of gray hairs in the site selected on the scalp of each subject belonging to the treatment group with every four weeks. In contrast subjects in the control group showed no visual reduction in gray hairs in the treatment site, or reduction in the density of gray hairs in the selected site on the scalp.

This study establishes that injectable administration of the injectable formulation of PAI-1 inhibitor reduces gray hair count and reverses hair graying in humans.

### Example 4: Effects of Topical PAI-1 inhibitor formulation on Keloids (not according to the claimed invention)

A topical study of topical PAI-1 inhibitor formulation after topical administration of a topical formulation of PAI-1 inhibitor (e.g., see Table 1) in man is performed. The study is designed to test whether the topical formulation of PAI-1 inhibitor significantly reduces keloids in man by measuring keloid size change following topical treatment with a PAI-1 inhibitor.

The study includes two groups of 25 human subjects each. Both groups have subjects with varying sizes and varying numbers of keloids. The keloid sites of each subject in the second group is treated twice a day for 6 months topically with a fixed volume of a PAI-1 inhibitor formulation that is at a fixed concentration of the PAI-1 inhibitor. The concentration of the PAI-1 inhibitor in the formulation is 5% w/w. The administration of the topical preparation to the scalp takes about 5 minutes, after which the suspension is left on the site for about 8 to 12 hours. The keloid sites of the subjects in the first group are treated twice a day for 6 months topically with an empty formulation and is the control.

The expected effect of such a treatment is a reduction in the size of keloids at the site of the PAI-1 inhibitor formulation treatment and/or a reduction in perceived pain. The reduction in size of keloid at the treatment sites is measured and a photograph of the treated area is taken to observe a change in the size of the keloid. The patient is also asked to score the perceived pain on a pain scale of 1-5 (1- no pain; 2 - little pain; 3 - moderate pain; 4 -severe pain; 5 - extremely severe pain).

A photograph and the pain scoring test methods are employed at baseline prior to a PAI-1 inhibitor treatment. Following this photographs of the keloid site of each subject is obtained every four weeks after start of treatment and at the end of the 6-month study; the pain score test is also performed every four weeks after start of treatment and at the end of the 6-month study. The study finds that at Baseline, the average size of the keloids as measured or as observed from the photographs is approximately equal across the control and treatment groups. Photographs of the keloid sites of the subjects of the treatment group on average show a visual reduction in the size of the keloids with every four weeks. The pain score test also on average shows a reduction in the in perceived pain at the site of each subject belonging to the treatment group with every four weeks. In contrast subjects in the control group showed no visual reduction in keloid size at the treatment site, or reduction in the in the perceived pain.

This study establishes that topical administration of the topical formulation of PAI-1 inhibitor treats keloids and reduces keloid size and perceived pain due to keloids in humans.

### Example 5: Effects of Oral PAI-1 inhibitor formulation on Keloids (not according to the claimed invention)

A study of oral administration of an oral formulation of PAI-1 inhibitor in man is performed. The study is designed to test whether the oral formulation of PAI-1 inhibitor significantly reduces keloids in man by measuring keloid size change following oral treatment with a PAI-1 inhibitor.

The study includes two groups of 25 human subjects each. Both groups have subjects with varying sizes and varying numbers of keloids. The keloid sites of each subject in the second group is administered an oral preparation of PAI-1 inhibitor formulated as a 75 mg capsule thrice a day for 6 months. Each subject in the first group are administered an oral empty preparation formulated as a 75 mg capsule thrice a day for 6 months and serve as the control.

The expected effect of such a treatment is a reduction in the size of keloids due to administration of the PAI-1 inhibitor formulation treatment and/or a reduction in perceived pain. The reduction in size of keloid is measured and a photograph of the treated area is taken to observe a change in the size of the keloid. The patient is also asked to score the perceived pain on a pain scale of 1-5 (1- no pain; 2 - little pain; 3 - moderate pain; 4 - severe pain; 5 - extremely severe pain).

A photograph and the pain scoring test methods are employed at baseline prior to a PAI-1 inhibitor treatment. Following this photographs of the keloid site of each subject is obtained every four weeks after start of treatment and at the end of the 6-month study; the pain score test is also performed every four weeks after start of treatment and at the end of the 6-month study. The study finds that at Baseline, the average size of the keloids as measured or as observed from the photographs is approximately equal across the control and treatment groups. Photographs of the keloid sites of the subjects of the treatment group on average show a visual reduction in the size of the keloids with every four weeks. The pain score test also on average shows a reduction in the in perceived pain at the site of each subject belonging to the treatment group with every four weeks. In contrast subjects in the control group showed no visual reduction in keloid size, or reduction in the in the perceived pain.

This study establishes that oral administration of the oral formulation of PAI-1 inhibitor treats keloids and reduces keloid size and perceived pain due to keloids in humans.

### Example 6: Effects of Injectable PAI-1 inhibitor formulation on Keloids (not according to the claimed invention)

A study of administration of an injectable formulation of PAI-1 inhibitor in man is performed. The study is designed to test whether the injectable formulation of PAI-1 inhibitor significantly reduces keloids in man by measuring keloid size change following injectable treatment with a PAI-1 inhibitor.

The study includes two groups of 25 human subjects each. Both groups have subjects with varying sizes and varying numbers of keloids. The keloid sites of each subject in the second group is treated twice a day for 6 months with a fixed volume of a PAI-1 inhibitor injectable formulation that is at a fixed concentration of the PAI-1 inhibitor. The concentration of the PAI-1 inhibitor in the formulation is 5% w/w. The administration of the injectable preparation to the keloid sites takes about 5 minutes, after which the suspension is left on the site for about 8 to 12 hours. The keloid sites of the subjects in the first group are treated twice a day for 6 months with an empty injectable formulation and is the control.

The expected effect of such a treatment is a reduction in the size of keloids due to the PAI-1 inhibitor formulation treatment and/or a reduction in perceived pain. The reduction in size of keloid at the treatment sites is measured and a photograph of the treated area is taken to observe a change in the size of the keloid. The patient is also asked to score the perceived pain on a pain scale of 1-5 (1- no pain; 2 - little pain; 3 - moderate pain; 4 - severe pain; 5 - extremely severe pain).

A photograph and the pain scoring test methods are employed at baseline prior to a PAI-1 inhibitor treatment. Following this photographs of the keloid site of each subject is obtained every four weeks after start of treatment and at the end of the 6-month study; the pain score test is also performed every four weeks after start of treatment and at the end of the 6-month study. The study finds that at Baseline, the average size of the keloids as measured or as observed from the photographs is approximately equal across the control and treatment groups. Photographs of the keloid sites of the subjects of the treatment group on average show a visual reduction in the size of the keloids with every four weeks. The pain score test also on average shows a reduction in the in perceived pain at the site of each subject belonging to the treatment group with every four weeks. In contrast subjects in the control group showed no visual reduction in keloid size at the treatment site, or reduction in the in the perceived pain.

This study establishes that administration of the injectable formulation of PAI-1 inhibitor treats keloids and reduces keloid size and perceived pain due to keloids in humans.

### Example 7: Effects of Topical PAI-1 inhibitor formulation on Scleroderma (not according to the claimed invention)

A topical study of topical PAI-1 inhibitor formulation after topical administration of a topical formulation of PAI-1 inhibitor (e.g., see Table 1) in man is performed. The study is designed to test whether the topical formulation of PAI-1 inhibitor significantly reduces one or more symptoms of cutaneous scleroderma (of the arms and legs) in man by measuring number of square centimeter (area) of scleroderma lesions following topical treatment with a PAI-1 inhibitor.

The study includes two groups of 25 human subjects each. Both groups have subjects with varying levels of cutaneous scleroderma (of the arms and legs) and varying numbers and/or density of scleroderma lesions. The scleroderma lesions of each subject in the second group is treated twice a day for 6 months topically with a fixed volume of a PAI-1 inhibitor formulation that is at a fixed concentration of the PAI-1 inhibitor. The concentration of the PAI-1 inhibitor in the formulation is 5% w/w. The administration of the topical preparation to the lesions takes about 5 minutes, after which the suspension is left on the site for about 8 to 12 hours. The sites of the subjects in the first group are treated twice a day for 6 months topically with an empty formulation and is the control.

The expected effect of such a treatment is a reduction in the number of square centimeter of scleroderma lesions at the site of the PAI-1 inhibitor formulation treatment. The number of square centimeter of scleroderma lesions at the treatment sites is measured by two methods: 1) A photograph of the treated area is taken to observe a change in number or lesions or size of lesions; or 2) A lesion area test, wherein the number of square centimeter of scleroderma lesions in a selected site on each subject is measured. The site to be measured in the subjects is selected prior to the commencement of the study.

A photograph and the lesion area test method are employed at baseline prior to a PAI-1 inhibitor treatment. Following this photographs of the site with lesions of each subject is obtained every four weeks after start of treatment and at the end of the 6-month study; the lesion area test is also performed every four weeks after start of treatment and at the end of the 6-month study. The study finds that at Baseline, the average amount of lesions observed from the photographs or average lesion area measured by the lesion area test is approximately equal across the control and treatment groups. Photographs of the sites of the subjects of the treatment group on average show a visual reduction in the area of the lesions with every four weeks. The lesion area test also on average shows a reduction in the number of square centimeter of scleroderma lesions in the site selected of each subject belonging to the treatment group with every four weeks. In contrast subjects in the control group showed no visual reduction in lesions in the treatment site, or reduction in the number of square centimeter of scleroderma lesions in the selected site.

This study establishes that topical administration of the topical formulation of PAI-1 inhibitor reduces one or more symptoms of cutaneous scleroderma (of the arms and legs) in humans.

### Example 8: Effects of Oral PAI-1 inhibitor formulation on Scleroderma (not according to the claimed invention)

A study of oral administration of an oral formulation of PAI-1 inhibitor in man is performed. The study is designed to test whether the oral formulation of PAI-1 inhibitor significantly reduces one or more symptoms of cutaneous scleroderma (of the arms and legs) in man by measuring number of square centimeter (area) of scleroderma lesions following oral treatment with a PAI-1 inhibitor.

The study includes two groups of 25 human subjects each. Both groups have subjects with varying levels of cutaneous scleroderma (of the arms and legs) and varying numbers and/or density of scleroderma lesions. Each subject in the second group is administered an oral preparation of PAI-1 inhibitor formulated as a 75 mg capsule thrice a day for 6 months. Each subject in the first group are administered an oral empty preparation formulated as a 75 mg capsule thrice a day for 6 months and serve as the control.

The expected effect of such a treatment is a reduction in the number of square centimeter of scleroderma lesions at the site of the PAI-1 inhibitor formulation treatment. The number of square centimeter of scleroderma lesions at the treatment sites is measured by two methods: 1) A photograph of the scalp is taken to observe a change in number or lesions or size of lesions; or 2) A lesion area test, wherein the number of square centimeter of scleroderma lesions in a selected site on each subject is measured. The site to be measured in the subjects is selected prior to the commencement of the study.

A photograph and the lesion area test method are employed at baseline prior to a PAI-1 inhibitor treatment. Following this photographs of the site with lesions of each subject is obtained every four weeks after start of treatment and at the end of the 6-month study; the lesion area test is also performed every four weeks after start of treatment and at the end of the 6-month study. The study finds that at Baseline, the average amount of lesions observed from the photographs or average lesion area measured by the lesion area test is approximately equal across the control and treatment groups. Photographs of the sites of the subjects of the treatment group on average show a visual reduction in the area of the lesions with every four weeks. The lesion area test also on average shows a reduction in the number of square centimeter of scleroderma lesions in the site selected of each subject belonging to the treatment group with every four weeks. In contrast subjects in the control group showed no visual reduction in lesions in the treatment site, or reduction in the number of square centimeter of scleroderma lesions in the selected site.

This study establishes that oral administration of the oral formulation of PAI-1 inhibitor reduces one or more symptoms of cutaneous scleroderma (of the arms and legs) in humans.

### Example 9: Effects of Injectable PAI-1 inhibitor formulation on Scleroderma (not according to the claimed invention)

A study of injectable PAI-1 inhibitor formulation after administration via injection of a sterile injectable formulation of PAI-1 inhibitor (e.g., see Table 1) in man is performed. The study is designed to test whether the injectable formulation of PAI-1 inhibitor significantly reduces one or more symptoms of cutaneous scleroderma (of the arms and legs) in man by number of square centimeter (area) of scleroderma lesions following treatment with a PAI-1 inhibitor.

The study includes two groups of 25 human subjects each. Both groups have subjects with varying levels of cutaneous scleroderma (of the arms and legs) and varying numbers and/or density of scleroderma lesions.. The scleroderma lesions of each subject in the second group is treated twice a day for 6 months with a fixed volume of a PAI-1 inhibitor injectable formulation that is at a fixed concentration of the PAI-1 inhibitor. The concentration of the PAI-1 inhibitor in the formulation is 5% w/w. The administration of the injectable preparation to the scalp takes about 5-10 minutes, after which the suspension is left on the site for about 8 to 12 hours. The sites of the subjects in the first group are treated twice a day for 6 months topically with an empty formulation and is the control.

The expected effect of such a treatment is a reduction in the number of square centimeter of scleroderma lesions at the site of the PAI-1 inhibitor formulation treatment. The number of square centimeter of scleroderma lesions at the treatment sites is measured by two methods: 1) A photograph of the treated area is taken to observe a change in number or lesions or size of lesions; or 2) A lesion area test, wherein the number of square centimeter of scleroderma lesions in a selected site on each subject is measured. The site to be measured in the subjects is selected prior to the commencement of the study.

A photograph and the lesion area test method are employed at baseline prior to a PAI-1 inhibitor treatment. Following this photographs of the site with lesions of each subject is obtained every four weeks after start of treatment and at the end of the 6-month study; the lesion area test is also performed every four weeks after start of treatment and at the end of the 6-month study. The study finds that at Baseline, the average amount of lesions observed from the photographs or average lesion area measured by the lesion area test is approximately equal across the control and treatment groups. Photographs of the sites of the subjects of the treatment group on average show a visual reduction in the area of the lesions with every four weeks. The lesion area test also on average shows a reduction in the number of square centimeter of scleroderma lesions in the site selected of each subject belonging to the treatment group with every four weeks. In contrast subjects in the control group showed no visual reduction in lesions in the treatment site, or reduction in the number of square centimeter of scleroderma lesions in the selected site.

This study establishes that injectable administration of the injectable formulation of PAI-1 inhibitor reduces one or more symptoms of cutaneous scleroderma (of the arms and legs) in humans.

### Example 10: Effects of Topical PAI-1 inhibitor formulation on Raynaud's Phenomenon (not according to the claimed invention)

A topical study of topical PAI-1 inhibitor formulation after topical administration of a topical formulation of PAI-1 inhibitor (e.g., see Table 1) in man is performed. The study is designed to test whether the topical formulation of PAI-1 inhibitor significantly reduces overall pain associated and number of Raynaud's pain episodes with Raynaud's disease in man by measuring overall pain associated with Raynaud's disease since last visit and number of Raynaud's pain episodes since last visit following topical treatment with a PAI-1 inhibitor.

The study includes two groups of 25 human subjects each. Both groups have subjects with varying levels overall pain associated with Raynaud's disease and number of Raynaud's pain episodes. The site affected by Raynaud's disease of each subject in the second group is treated twice a day for 6 months topically with a fixed volume of a PAI-1 inhibitor formulation that is at a fixed concentration of the PAI-1 inhibitor. The concentration of the PAI-1 inhibitor in the formulation is 5% w/w. The administration of the topical preparation to the affected site takes about 5 minutes, after which the suspension is left on the site for about 8 to 12 hours. The affected sites of the subjects in the first group are treated twice a day for 6 months topically with an empty formulation and is the control.

The expected effect of such a treatment is a reduction in the overall pain associated with Raynaud's disease since last visit and number of Raynaud's pain episodes since last visit at the site of the PAI-1 inhibitor formulation treatment. The overall pain associated with Raynaud's disease since last visit and number of Raynaud's pain episodes since last visit at the treatment sites is measured by: 1) A pain scale of 1-5 (1- no pain; 2 - little pain; 3 - moderate pain; 4 -severe pain; 5 - extremely severe pain); and/or 2) Number of Raynaud's pain episodes at the treatment site for each subject.

The subjects record the overall pain associated with Raynaud's disease and number of Raynaud's pain episodes experienced over the last four weeks at baseline prior to a PAI-1 inhibitor treatment. Following the overall pain associated with Raynaud's disease since last visit and number of Raynaud's pain episodes since last visit of each subject is obtained every four weeks after start of treatment and at the end of the 6-month study. The study finds that at Baseline, the average amount of overall pain associated with Raynaud's disease and number of Raynaud's pain episodes is approximately equal across the control and treatment groups. On average a reduction in overall Raynaud's pain scores and number of Raynaud's pain episodes since last visit is observed in the subjects of the treatment group with every four weeks. In contrast subjects in the control group showed no reduction in overall pain score since last visit, or reduction in the number of Raynaud's pain episodes since last visit.

This study establishes that topical administration of the topical formulation of PAI-1 inhibitor reduces overall pain associated and number of Raynaud's pain episodes with Raynaud's disease in humans.

### Example 11: Effects of Oral PAI-1 inhibitor formulation on Raynaud's Phenomenon (not according to the claimed invention)

A study of oral administration of an oral formulation of PAI-1 inhibitor in man is performed. The study is designed to test whether the oral formulation of PAI-1 inhibitor significantly reduces overall pain associated and number of Raynaud's pain episodes with Raynaud's disease in man by measuring overall pain associated with Raynaud's disease since last visit and number of Raynaud's pain episodes since last visit following oral treatment with a PAI-1 inhibitor.

The study includes two groups of 25 human subjects each. Both groups have subjects with varying levels overall pain associated with Raynaud's disease and number of Raynaud's pain episodes. Each subject in the second group is administered an oral preparation of PAI-1 inhibitor formulated as a 75 mg capsule thrice a day for 6 months. Each subject in the first group are administered an oral empty preparation formulated as a 75 mg capsule thrice a day for 6 months and serve as the control.

The expected effect of such a treatment is a reduction in the overall pain associated with Raynaud's disease since last visit and number of Raynaud's pain episodes since last visit in subjects following the PAI-1 inhibitor formulation treatment. The overall pain associated with Raynaud's disease since last visit and number of Raynaud's pain episodes since last visit at the treatment sites is measured by: 1) A pain scale of 1-5 (1- no pain; 2 - little pain; 3 - moderate pain; 4 -severe pain; 5 - extremely severe pain); and/or 2) Number of Raynaud's pain episodes at the treatment site for each subject.

The subjects record the overall pain associated with Raynaud's disease and number of Raynaud's pain episodes experienced over the last four weeks at baseline prior to a PAI-1 inhibitor treatment. Following the overall pain associated with Raynaud's disease since last visit and number of Raynaud's pain episodes since last visit of each subject is obtained every four weeks after start of treatment and at the end of the 6-month study. The study finds that at Baseline, the average amount of overall pain associated with Raynaud's disease and number of Raynaud's pain episodes is approximately equal across the control and treatment groups. On average a reduction in overall Raynaud's pain scores and number of Raynaud's pain episodes since last visit is observed in the subjects of the treatment group with every four weeks. In contrast subjects in the control group showed no reduction in overall pain score since last visit, or reduction in the number of Raynaud's pain episodes since last visit.

This study establishes that oral administration of the oral formulation of PAI-1 inhibitor reduces overall pain associated and number of Raynaud's pain episodes with Raynaud's disease in humans.

### Example 12: Effects of Injectable PAI-1 inhibitor formulation on Raynaud's Phenomenon (not according to the claimed invention)

A study of injectable PAI-1 inhibitor formulation after administration via injection of a sterile injectable formulation of PAI-1 inhibitor (e.g., see Table 1) in man is performed. The study is designed to test whether the injectable formulation of PAI-1 inhibitor significantly reduces overall pain associated and number of Raynaud's pain episodes with Raynaud's disease in man by measuring overall pain associated with Raynaud's disease since last visit and number of Raynaud's pain episodes since last visit following treatment with a PAI-1 inhibitor.

The study includes two groups of 25 human subjects each. Both groups have subjects with varying levels overall pain associated with Raynaud's disease and number of Raynaud's pain episodes. The site affected by Raynaud's disease of each subject in the second group is treated twice a day for 6 months with a fixed volume of a PAI-1 inhibitor injectable formulation that is at a fixed concentration of the PAI-1 inhibitor. The concentration of the PAI-1 inhibitor in the formulation is 5% w/w. The administration of the injectable preparation to the site takes about 5-10 minutes, after which the suspension is left on the site for about 8 to 12 hours. The sites of the subjects in the first group are treated twice a day for 6 months topically with an empty formulation and is the control.

The expected effect of such a treatment is a reduction in the overall pain associated with Raynaud's disease since last visit and number of Raynaud's pain episodes since last visit at the site of the PAI-1 inhibitor formulation treatment. The overall pain associated with Raynaud's disease since last visit and number of Raynaud's pain episodes since last visit at the treatment sites is measured by: 1) A pain scale of 1-5 (1- no pain; 2 - little pain; 3 - moderate pain; 4 -severe pain; 5 - extremely severe pain); and/or 2) Number of Raynaud's pain episodes at the treatment site for each subject.

The subjects record the overall pain associated with Raynaud's disease and number of Raynaud's pain episodes experienced over the last four weeks at baseline prior to a PAI-1 inhibitor treatment. Following the overall pain associated with Raynaud's disease since last visit and number of Raynaud's pain episodes since last visit of each subject is obtained every four weeks after start of treatment and at the end of the 6-month study. The study finds that at Baseline, the average amount of overall pain associated with Raynaud's disease and number of Raynaud's pain episodes is approximately equal across the control and treatment groups. On average a reduction in overall Raynaud's pain scores and number of Raynaud's pain episodes since last visit is observed in the subjects of the treatment group with every four weeks. In contrast subjects in the control group showed no reduction in overall pain score since last visit, or reduction in the number of Raynaud's pain episodes since last visit.

This study establishes that injectable administration of the injectable formulation of PAI-1 inhibitor reduces overall pain associated and number of Raynaud's pain episodes with Raynaud's disease in humans.

## Claims

1. A non-therapeutic cosmetic process for treating or preventing hair graying, the process comprising providing a composition that comprises a plasminogen activator inhibitor-1 (PAI-1) inhibitor and:
(i) administering the composition to a site of a subject, wherein the site contains or did contain a plurality of hair follicles, each with a hair disposed therein, so that the PAI-1 inhibitor is delivered to the subject; or
(ii) administering the composition to a subject, wherein a site of the subject contains or did contain a plurality of hair follicles, each with a hair disposed therein, so that the PAI-1 inhibitor is delivered to the subject;
wherein the PAI-1 inhibitor is selected from 5-Chloro-2-{[(2-{[3-(furan-3-yl)phenyl]amino}-2-oxoethoxy)acetyl]amino benzoic acid, and 5-Chloro-2-{[{[3-(furan-3-yl)phenyl]amino}(oxo)acetyl]amino} benzoic acid.

2. The process of claim 1, wherein the administering is:
(i) by topically applying to a site on a skin surface, preferably wherein the administering comprises maintaining the composition on the skin surface for a period of time, preferably further comprising, after the period of time, removing remaining composition from the site; preferably wherein the period of time is at least 1 minute; optionally wherein the period of time is within a range of 1 to 10 minutes; or wherein the period of time is at least 1 hour; or
(ii) by injection; or
(iii) by oral administration.

3. The process of claim 2, wherein the administering is (i) by topically applying to a site on a skin surface or (ii) by injection, and wherein the step of administering the composition comprises massaging the composition into the site.

4. The process of any one of the preceding claims, wherein the PAI-1 inhibitor is 5-Chloro-2-{[(2-{[3-(furan-3-yl)phenyl]amino}-2-oxoethoxy)acetyl]amino benzoic acid.

5. The process of any one of the preceding claims, wherein the composition comprising the PAI-1 inhibitor is or comprises a suspension, or wherein the composition comprising the PAI-1 inhibitor is or comprises a foam, or wherein the composition comprising the PAI-1 inhibitor comprises an emulsion, preferably wherein the emulsion is a nanoemulsion.

6. The process of any one of the preceding claims, wherein the administering is i) by topically applying to a site on a skin surface, further comprising a step of administering a penetrating treatment, preferably wherein the penetrating treatment is or comprises a non-irritating chemical agent, or wherein the penetrating treatment is or comprises administration of an electric or magnetic field, or wherein the penetrating treatment is or comprises microneedling, or wherein the penetrating treatment is or comprises laser treatment.

7. The process of any one of the preceding claims, comprising more than one administration of the composition over time, preferably wherein each administration comprising the PAI-1 inhibitor is separated by a specified period of time.

8. The process of any one of the preceding claims, wherein the administering is by topically applying to a site on a skin surface, and wherein the composition is formulated as a suspension, a foam, a lotion, a cream, a gel, an oil, a powder, a liniment, or drops.

9. The process of any one of the preceding claims, wherein the process further comprises administering one or more other active agents, wherein the one or more other active agents is selected from the group comprising of cinnamidopropyltrimonium chloride, solid lipid nanoparticles, I-cystine, I-methionine, melatonin, and combinations thereof.

## Patentansprüche

1. Nicht-therapeutisches kosmetisches Verfahren für das Behandeln oder Verhindern des Ergrauens der Haare, das Verfahren umfassend das Bereitstellen einer Zusammensetzung, die einen Inhibitor des Plasminogenaktivator-Inhibitors-1 (PAI-1) umfasst, und:
(i) Verabreichen der Zusammensetzung an einer Stelle eines Subjekts, wobei die Stelle eine Vielzahl von Haarfollikeln enthält oder enthielt, jedes mit einem darin angeordneten Haar, sodass der PAI-1-Inhibitor dem Subjekt zugeführt wird; oder
(ii) Verabreichen der Zusammensetzung an ein Subjekt, wobei eine Stelle des Subjekts eine Vielzahl von Haarfollikeln enthält oder enthielt, jedes mit einem darin angeordneten Haar, sodass der PAI-1-Inhibitor dem Subjekt zugeführt wird;
wobei der PAI-1-Inhibitor ausgewählt ist aus 5-Chlor-2-{[(2-{[3-(furan-3-yl)phenyl]amino}-2-oxoethoxy)acetyl]amino}benzoesäure und 5-Chlor-2-{[{[3-(furan-3-yl)phenyl]amino}(oxo)acetyl]amino}benzoesäure.

2. Verfahren nach Anspruch 1, wobei sich das Verabreichen wie folgt darstellt:
(i) durch topisches Auftragen auf eine Stelle der Hautoberfläche, vorzugsweise wobei das Verabreichen das Belassen der Zusammensetzung auf der Hautoberfläche für eine bestimmte Zeitspanne umfasst, vorzugsweise ferner umfassend, nach Ablauf der Zeitspanne, das Entfernen der verbleibenden Zusammensetzung von der Stelle; vorzugsweise wobei die Zeitspanne mindestens 1 Minute beträgt; optional wobei die Zeitspanne in einem Bereich von 1 bis 10 Minuten liegt oder wobei die Zeitspanne mindestens eine Stunde beträgt; oder
(ii) durch Injizieren; oder
(iii) durch orale Verabreichung.

3. Verfahren nach Anspruch 2, wobei das Verabreichen (i) durch topisches Auftragen auf eine Stelle auf der Hautoberfläche oder (ii) durch Injektion erfolgt, und wobei der Schritt des Verabreichens der Zusammensetzung das Einmassieren der Zusammensetzung in die Stelle umfasst.

4. Verfahren nach einem der vorstehenden Ansprüche, wobei der PAI-1-Inhibitor 5-Chlor-2-{[(2-{[3-(furan-3-yl)phenyl]amino}-2-oxoethoxy)acetyl]aminobenzoesäure ist.

5. Verfahren nach einem der vorstehenden Ansprüche, wobei die Zusammensetzung, die den PAI-1-Inhibitor umfasst, eine Suspension ist oder umfasst, oder wobei die Zusammensetzung, die den PAI-1-Inhibitor umfasst, ein Schaum ist oder einen solchen umfasst, oder wobei die Zusammensetzung, die den PAI-1-Inhibitor umfasst, eine Emulsion umfasst, vorzugsweise wobei die Emulsion eine Nanoemulsion ist.

6. Verfahren nach einem der vorstehenden Ansprüche, wobei das Verabreichen (i) durch topisches Auftragen auf eine Stelle auf einer Hautoberfläche erfolgt, ferner umfassend einen Schritt zum Verabreichen einer tief eindringenden Behandlung, vorzugsweise wobei die tief eindringende Behandlung ein nicht reizender chemischer Wirkstoff ist oder einen solchen umfasst, oder wobei die tief eindringende Behandlung eine Verabreichung eines elektrischen oder magnetischen Feldes ist oder ein solches umfasst, oder wobei die tief eindringende Behandlung Microneedling ist oder ein solches umfasst, oder wobei die tief eindringende Behandlung eine Laserbehandlung ist oder eine solche umfasst.

7. Verfahren nach einem der vorstehenden Ansprüche, umfassend mehr als eine Verabreichung der Zusammensetzung über die Zeit, vorzugsweise wobei jede Verabreichung, die den PAI-1-Inhibitor umfasst, durch eine bestimmte Zeitspanne getrennt ist.

8. Verfahren nach einem der vorstehenden Ansprüche, wobei das Verabreichen durch topisches Auftragen auf eine Stelle auf einer Hauptoberfläche erfolgt, und wobei die Zusammensetzung die Formel einer Suspension, eines Schaums, einer Lotion, einer Creme, eines Gels, eines Öls, eines Pulvers, einer Salbe oder von Tropfen aufweist.

9. Verfahren nach einem der vorstehenden Ansprüche, wobei das Verfahren ferner das Verabreichen eines oder mehrerer Wirkstoffe umfasst, wobei der eine oder die mehreren Wirkstoffe ausgewählt sind aus der Gruppe bestehend aus Cinnamidopropyltrimoniumchlorid, festen Lipidnanopartikeln, L-Cystin, L-Methionin, Melatonin und Kombinationen davon.

## Revendications

1. Procédé cosmétique non thérapeutique pour le traitement ou la prévention de canitie, le procédé comprenant la fourniture d'une composition qui comprend un inhibiteur inhibiteur-1 de l'activateur du plasminogène (Plasminogen Activator Inhibitor-1, PAI-1) et :
(i) l'administration de la composition à un site d'un sujet, dans lequel le site contient ou contenait une pluralité de follicules, chacun avec un cheveu disposé dans celui-ci, de telle sorte que l'inhibiteur PAI-1 est administré au sujet ; ou
(ii) l'administration de la composition à un sujet, dans lequel un site du sujet contient ou contenait une pluralité de follicules, chacun avec un cheveu disposé dans celui-ci, de telle sorte que l'inhibiteur PAI-1 est administré au sujet ;
dans lequel l'inhibiteur PAI-1 est sélectionné parmi l'acide 5-chloro-2-{[(2-{[3-(furan-3-yl)phényl]amino}-2-oxoéthoxy)acétyl]aminobenzoïque, et l'acide 5-chloro-2-{[{[3-(furan-3-yl)phényl]amino}(oxo)acétyl]amino}benzoïque.

2. Procédé de la revendication 1, dans lequel l'administration se fait :
(i) par application topique sur un site sur une surface cutanée, de préférence dans lequel l'administration comprend le maintien de la composition sur la surface cutanée pendant un laps de temps, de préférence comprenant en outre, après le laps de temps, l'élimination de la composition restante du site ; de préférence, dans lequel le laps de temps est d'au moins 1 minute ; facultativement, dans lequel le laps de temps est dans les limites d'une plage de 1 à 10 minutes ; ou dans lequel le laps de temps est d'au moins 1 heure ; ou
(ii) par injection ; ou
(iii) par administration orale.

3. Procédé de la revendication 2, dans lequel l'administration se fait (i) par application topique sur un site sur une surface cutanée ou (ii) par injection, et dans lequel l'étape de l'administration de la composition comprend le massage de la composition dans le site.

4. Procédé de l'une quelconque des revendications précédentes, dans lequel l'inhibiteur PAI-1 est l'acide 5-chloro-2-{[(2-{[3-(furan-3-yl)phényl]amino}-2-oxoéthoxy)acétyl]aminobenzoïque.

5. Procédé de l'une quelconque des revendications précédentes, dans lequel la composition comprenant l'inhibiteur PAI-1 est ou comprend une suspension, ou dans lequel la composition comprenant l'inhibiteur PAI-1 est ou comprend une mousse, ou dans lequel la composition comprenant l'inhibiteur PAI-1 comprend une émulsion, de préférence dans lequel l'émulsion est une nano-émulsion.

6. Procédé de l'une quelconque des revendications précédentes, dans lequel l'administration se fait i) par application topique sur un site sur une surface cutanée, comprenant en outre une étape de l'administration d'un traitement pénétrant, de préférence dans lequel le traitement pénétrant est ou comprend un agent chimique non irritant, ou dans lequel le traitement pénétrant est ou comprend l'administration d'un champ électrique ou magnétique, ou dans lequel le traitement pénétrant est ou comprend le micro-aiguillage, ou dans lequel le traitement pénétrant est ou comprend un traitement laser.

7. Procédé de l'une quelconque des revendications précédentes, comprenant plus d'une administration de la composition au fil du temps, de préférence dans lequel chaque administration comprenant l'inhibiteur PAI-1 est séparée par un laps de temps spécifié.

8. Procédé de l'une quelconque des revendications précédentes, dans lequel l'administration se fait par application topique sur un site sur une surface cutanée, et dans lequel la composition est préparée sous forme de suspension, de mousse, de lotion, de crème, de gel, d'huile, de poudre, de liniment, ou de gouttes.

9. Procédé de l'une quelconque des revendications précédentes, dans lequel le procédé comprend en outre l'administration d'un ou de plusieurs autres agents actifs, dans lequel l'un ou les plusieurs autres agents actifs sont sélectionnés parmi le groupe composé de : chlorure de cinnamidopropyltrimonium, nanoparticules lipidiques solides, I-cystine, I-méthionine, mélatonine, et combinaisons de ceux-ci.
